# EUROPEAN PATENT APPLICATION

(11) **EP 4 335 876 A1**
(43) Date of publication of application: **13.03.2024**
(21) Application number: 23194941.3
(22) Date of filing: 01.09.2023
(51) Int. Cl.: C08B 37/08, A61K 31/728, C08J 3/075, C08L 5/08

(54) **METHOD OF MANUFACTURING AUTO-CROSSLINKED HYALURONIC ACID GEL AND PRODUCTS THEREOF**

(30) Priority: 06.09.2022 TW 111133781
(71) Applicant: Scivision Biotech Inc., Kaohsiung City 806 (TW)
(72) Inventor: HAN, TAI-SHIEN, 806 KAOHSIUNG CITY (TW); PAN, TSUNG-WEI, 806 KAOHSIUNG CITY (TW); CHEN, TOR-CHERN, 806 KAOHSIUNG CITY (TW); CHEN, CHUN-CHANG, 806 KAOHSIUNG CITY (TW); LIN, PO-HSUAN, 806 KAOHSIUNG CITY (TW); CHEN, LI-SU, 806 KAOHSIUNG CITY (TW)
(74) Representative: 2K Patentanwälte Blasberg Kewitz & Reichel

(57) **Abstract**

The present disclosure provides a method for manufacturing an auto-crosslinked hyaluronic acid gel, comprising conducting auto-crosslinking reaction of a colloid containing hyaluronic acid continuously at low temperature in an acidic environment, and treating the reaction product with steam at high temperature to obtain the auto-crosslinked hyaluronic acid gel with high viscosity.

## Description

### FIELD OF THE INVENTION

The present disclosure relates to a method of manufacturing hyaluronic acid, particularly of auto-crosslinked hyaluronic acid gel and a product thereof.

### BACKGROUND OF THE INVENTION

Hyaluronic acid is a linear polysaccharide formed from disaccharide units having a molecular weight of about 400 D, and composed ofβ-1,3-N-acetyl glucosamine andβ-1, 4-glucuronic acid, linked via alternating β-1-4 and β-1-3 glycosidic bonds. Commercial hyaluronic acid may be extracted from fermentation of bacteria, such as *Streptococcus,* or from animal tissues, such as rooster combs. Linear hyaluronic acid is rapidly degraded *in vivo* by certain enzymes (such as hyaluronidases) or free-radical reaction, thus reducing its half-life *in vivo.* In addition, the application of linear hyaluronic acid is limited due to the lack of mechanical strength. Accordingly, hyaluronic acid is often crosslinked for practical applications.

Auto-crosslinked hyaluronic acid is an ester produced by the esterification of inter- or intramolecular hydroxyl groups (-OH) and carboxyl groups (-COOH) of hyaluronic acid in the presence of an activator or catalyst. Since no crosslinking agent is used, no molecular fragments of crosslinking groups are formed on the resulting auto-crosslinked hyaluronic acid, which can, accordingly, retain superior biocompatibility and viscoelastic properties, and has been widely used in various medical applications. For example, the auto-crosslinked hyaluronic acid may be applied for treatment of arthropathy (e.g., as disclosed in US 6,251,876), prevention of post-operative adhesion (e.g., US 7,504,386), or as subcutaneous fillers (e.g., as disclosed in Andrea Alessandrini et al, Plastic and Reconstructive Surgery, vol. 118, pp. 341-346 (2006)). The auto-crosslinked hyaluronic acid is also widely used in tissue engineering applications. Auto-crosslinked hyaluronic acid based post-operative anti-adhesive gels are commercially available as Hyalobarrier^{®} and HYALOGLIDE^{®}.

### SUMMARY OF THE INVENTION

To address one or more of the foregoing issues, the present disclosure provides a low-temperature auto-crosslinking reaction process in the presence of acid for producing a high-viscosity auto-crosslinked hyaluronic acid gel that is steam-treated.

The preset disclosure provides a method for manufacturing an auto-crosslinked hyaluronic acid gel, comprising steps of:
(a) providing a colloid containing hyaluronic acid or a metal salt thereof;
(b) conducting an auto-crosslinking reaction of the colloid containing hyaluronic acid or a metal salt thereof in an acidic environment at about -10°C to about -30°C for about 7 days to about 42 days; and
(c) treating the product of step (b) by steam to obtain the auto-crosslinked hyaluronic acid gel.

Examples of the salt of hyaluronic acid may be an alkali metal salt include, but are not limited to, potassium, sodium, zinc, or lithium salts of hyaluronic acid. For example, the metal salt of hyaluronic acid may be sodium hyaluronate.

In some embodiments of the present disclosure, the method is free of using any crosslinking agents.

In some embodiments of the present disclosure, step (a) comprises providing the colloid containing hyaluronic acid or a metal salt thereof in a granule form, a stripe form or a string form.

In some embodiments of the present disclosure, in step (b), the acidic environment is provided by an acid at a concentration of about 0.5 N to about 1.5 N, such as of about 0.51 N to about 1.4 N, of about 0.52 N to about 1.3 N, of about 0.53 N to about 1.2 N, of about 0.54 N to about 1.1 N, of about 0.55 N to about 1.0 N, of about 0.56 N to about 0.9 N, of about 0.57 N to about 0.8 N, of about 0.58 N to about 0.7 N, or of about 0.59 N to about 0.6 N. In some embodiments of the present disclosure, the acid is hydrochloric acid, nitric acid and/or sulfuric acid.

In some embodiments of the present disclosure, step (b) comprises mixing the colloid containing hyaluronic acid or a metal salt thereof with an acid at room temperature to form a mixture, and letting the mixture stand at about -10°C to about -30°C for about 7 days to about 42 days. For example, the mixture stands at about -12°C to about -28°C, at about -14°C to about -26°C, at about -16°C to about -24°C, at about -18°C to about -22°C, at about -10°C, at about - 20°C, or at about -30°C; for about 9 to about 40 days, about 11 to about 40 days, about 13 to about 38 days, about 15 to about 36 days, about 17 to about 34 days, about 19 to about 32 days, about 21 to about 30 days, about 23 to about 28 days, about 25 to about 27 days, about 7 days, about 14 days, about 21 days, or about 28 days. In some embodiments of the present disclosure, a concentration of hyaluronic acid or a metal salt thereof in the mixture is about 10 wt% to about 30 wt%, such as about 11 wt% to about 28 wt%, about 12 wt% to about 26 wt%, about 13 wt% to about 24 wt%, about 14 wt% to about 22 wt%, about 15 wt% to about 20 wt%, about 16 wt% to about 18 wt%, about 10 wt% to about 20 wt%, or about 10 wt% to about 14.5 wt%.

In some embodiments of the present disclosure, in step (b),
the acidic environment is provided by an acid at a concentration of about 0.58 N to about 1.0 N, and the auto-crosslinking reaction is conducted at about -10°C for about 7 days to about 42 days;
the acidic environment is provided by an acid at a concentration of about 0.58 N to about 1.5 N, and the auto-crosslinking reaction is conducted at about -20°C for about 7 days to about 35 days;
the acidic environment is provided by an acid at a concentration of about 0.58 N to about 1.0 N, and the auto-crosslinking reaction is conducted at about -20°C for about 7 days to about 28 days;
the acidic environment is provided by an acid at a concentration of about 1.0 N to about 1.5 N, and the auto-crosslinking reaction is conducted at about -30°C for about 14 days to about 35 days; or
the acidic environment is provided by an acid at a concentration of about 0.58 N to about 1.0 N, and the auto-crosslinking reaction is conducted at about -30°C for about 14 days to about 35 days.

In some embodiments of the present disclosure, the method comprises conducting the auto-crosslinking reaction for only one time, and the entire auto-crosslinking reaction is conducted continuously at about -10°C to about -30°C.

In some embodiments of the present disclosure, before step (c), the method further comprises homogenizing and/or washing the product of step (b).

In some embodiments of the present disclosure, the product of step (b) has an equilibrium swelling capacity of about 12 to about 30.

In some embodiments of the present disclosure, before step (c), the method further comprises adjusting a pH of the product of step (b) to about 6.0 to about 7.0. In some embodiments of the present disclosure, before step (c), the method further comprises adjusting a pH of the product of step (b) to about 6.3 to about 6.8. In some embodiments of the present disclosure, the pH adjusting step is after the homogenizing and/or washing step.

The temperature and time of the steam treatment is not limited in the present disclosure, provided that the auto-crosslinked hyaluronic acid gel of the present disclosure can be successfully produced. In some embodiments of the present disclosure, step (c) comprises treating the product of step (b) by steam at about 110°C to about 130°C for about 10 minutes to about 60 minutes. For example, the temperature of the steam used in step (c) is about 112°C to about 128°C, about 114°C to about 126°C, about 116°C to about 124°C, about 118°C to about 122°C, or about 121°C.

In some embodiments of the present disclosure, the method is for manufacturing the auto-crosslinked hyaluronic acid gel the auto-crosslinked hyaluronic acid gel exhibits at least one of:
a tan δ of the auto-crosslinked hyaluronic acid gel at a concentration of about 1.5 wt% to about 7.5 wt% measured at 25°C and at each and every frequency between 0.1 Hz to 10.0 Hz is equal to or less than 1;
a modulus of elasticity of the auto-crosslinked hyaluronic acid gel at a concentration of about 1.5 wt% to about 7.5 wt% measured at 25°C and at 1.0 Hz is about 30 Pa to about 8000 Pa;
a modulus of viscosity of the auto-crosslinked hyaluronic acid gel at a concentration of about 1.5 wt% to about 7.5 wt% measured at 25°C and at 1.0 Hz is about 20 Pa to about 2000 Pa; or
a shear viscosity of the auto-crosslinked hyaluronic acid gel at a concentration of about 1.5 wt% to about 7.5 wt% measured at 25°C and at a shear rate of 1 sec⁻¹ is about 20 Pa·s to about 900 Pa·s. In some embodiments of the present disclosure, the aforementioned concentration refers to the concentration of hyaluronic acid of a salt thereof in the auto-crosslinked hyaluronic acid gel.

The present disclosure further provides an auto-crosslinked hyaluronic acid gel, wherein a tan δ of the auto-crosslinked hyaluronic acid gel at a concentration of about 1.5 wt% to about 7.5 wt% measured at 25°C and at each and every frequency between 0.1 Hz to 10.0 Hz is equal to or less than 1, and the auto-crosslinked hyaluronic acid gel is sterile.

In some embodiments of the present disclosure, a modulus of elasticity of the auto-crosslinked hyaluronic acid gel at a concentration of about 1.5 wt% to about 7.5 wt% measured at 25°C and at 1.0 Hz is about 30 Pa to about 8000 Pa.

In some embodiments of the present disclosure, a modulus of viscosity of the auto-crosslinked hyaluronic acid gel at a concentration of about 1.5 wt% to about 7.5 wt% measured at 25°C and at 1.0 Hz is about 20 Pa to about 2000 Pa.

In some embodiments of the present disclosure, a shear viscosity of the auto-crosslinked hyaluronic acid gel at a concentration of about 1.5 wt% to about 7.5 wt% measured at 25°C and at a shear rate of 1 sec⁻¹ is about 20 Pa·s to about 900 Pa·s, about 38.04 Pa·s to about 896.91 Pa·s, or about 55.69 Pa·s to about 896.91 Pa·s.

In some embodiments of the present disclosure, a shear viscosity of the auto-crosslinked hyaluronic acid gel at a concentration of about 1.5 wt% to about 7.5 wt% measured at 25°C and at a shear rate of 50 sec⁻¹ is about 0.31 Pa·s to about 35.66 Pa·s, about 1.97 Pa·s to about 35.66 Pa·s, or about 2.18 Pa·s to about 35.66 Pa·s.

The present disclosure further provides a method for promoting bone regeneration, treating bone defects, preventing post-operative adhesion, subcutaneous filling and/or treating arthropathy in a subject in need thereof, comprising administering an auto-crosslinked hyaluronic acid gel to the subject in need.

### BRIEF DESCRIPTION OF DRAWINGS

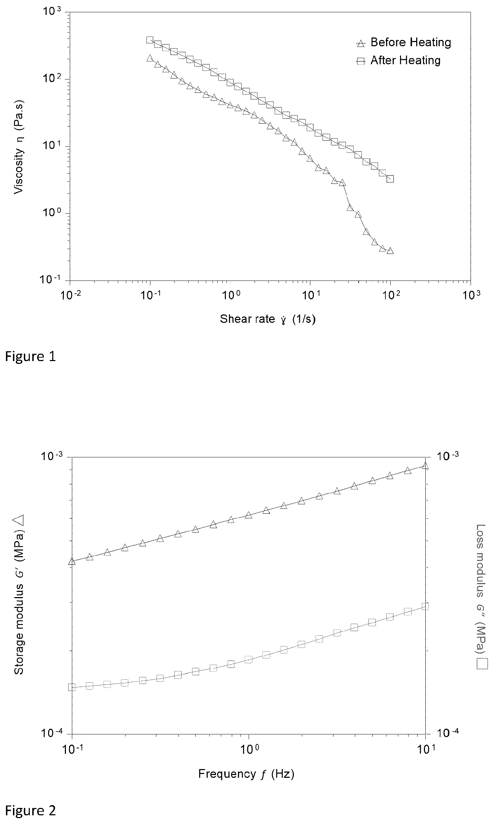

FIG. 1 shows a comparison of shear viscosities of the auto-crosslinked hyaluronic acid particles (before steam treatment) and the auto-crosslinked hyaluronic acid gel (after steam treatment). The auto-crosslinked hyaluronic acid particles is prepared by auto-crosslinking reaction of colloid containing hyaluronic acid or a salt thereof (hyaluronic acid concentration 14.5 wt %) in the presence of 1.0 N HCl at -10°C for14 days. The pH of the auto-crosslinked hyaluronic acid particles (before steam treatment) is 6.5, and the auto-crosslinked hyaluronic acid particles is treated by steam at 121°C for 15 minutes.
FIG. 2 shows a modulus of elasticity (G') and modulus of viscosity (G") of the auto-crosslinked hyaluronic acid gel versus frequency of measurement. The auto-crosslinked hyaluronic acid particles is prepared by auto-crosslinking reaction of colloid containing hyaluronic acid or a salt thereof (hyaluronic acid concentration 14.5 wt %) in the presence of 1.5 N HCl at -30°C for14 days. The pH of the auto-crosslinked hyaluronic acid particles (before steam treatment) is 6.5, and the auto-crosslinked hyaluronic acid particles is treated by steam at 121°C for 15 minutes.

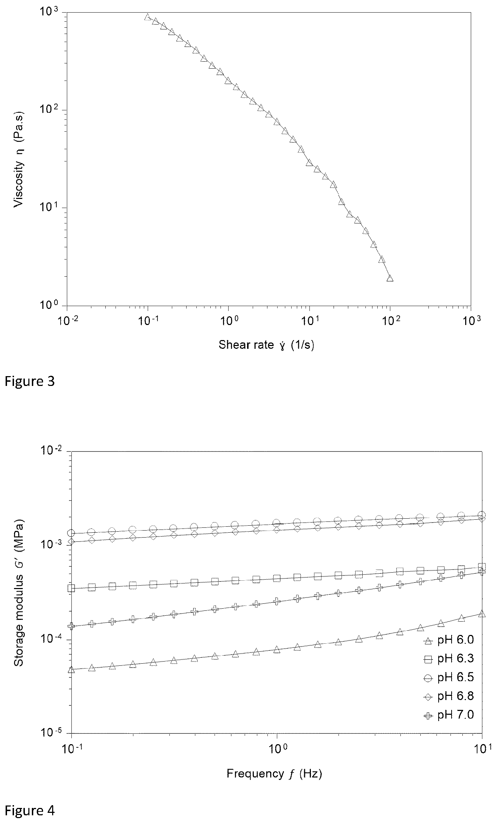

FIG. 3 shows shear viscosity of the auto-crosslinked hyaluronic acid gel versus shear rate. The auto-crosslinked hyaluronic acid particles is prepared by auto-crosslinking reaction of colloid containing hyaluronic acid or a salt thereof (hyaluronic acid concentration 14.5 wt %) in the presence of 1.5 N HCl at -30°C for 14 days. The pH of the auto-crosslinked hyaluronic acid particles (before steam treatment) is 6.5, and the auto-crosslinked hyaluronic acid particles is treated by steam at 121°C for 15 minutes.
FIG. 4 shows the effect of pH of the auto-crosslinked hyaluronic acid particles (before steam treatment) on modulus of elasticity of the auto-crosslinked hyaluronic acid gel (after steam treatment). The auto-crosslinked hyaluronic acid particles is prepared by auto-crosslinking reaction of colloid containing hyaluronic acid or a salt thereof (hyaluronic acid concentration 14.5 wt %) in the presence of 1.5 N HCl at -20°C for 21 days. The auto-crosslinked hyaluronic acid particles is treated by steam at 121°C for 20 minutes.

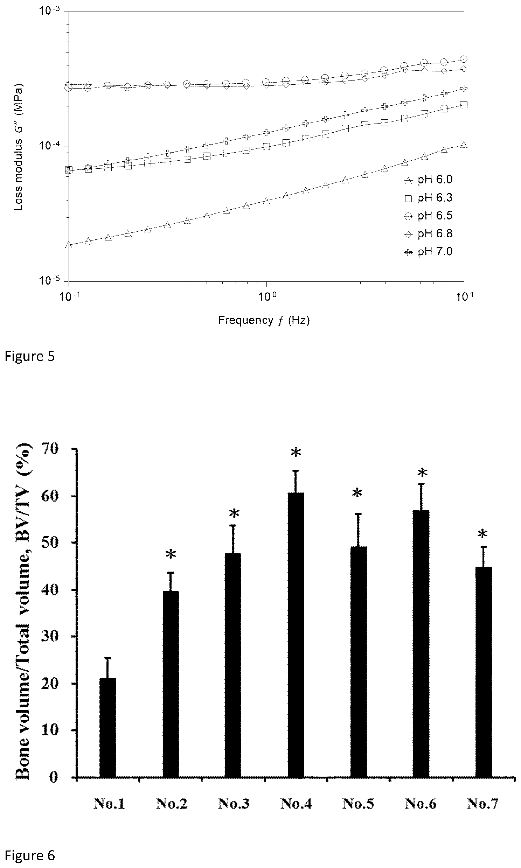

FIG. 5 shows the effect of pH of the auto-crosslinked hyaluronic acid particles (before steam treatment) on modulus of viscosity of the auto-crosslinked hyaluronic acid gel (after steam treatment). The auto-crosslinked hyaluronic acid particles is prepared by auto-crosslinking reaction of colloid containing hyaluronic acid or a salt thereof (hyaluronic acid concentration 14.5 wt %) in the presence of 1.5 N HCl at -20°C for 21 days. The auto-crosslinked hyaluronic acid particles is treated by steam at 121°C for 20 minutes.
FIG. 6 shows experimental results comparing percentages of newly formed bone after cranial defect in terms of volume following treatment with the disclosed auto-crosslinked hyaluronic acid gel and control groups, corresponding to Table 14. No. 1: blank group: after cranial defect surgery, the wound is directly covered with Bio-Gide membrane; No. 2: after cranial defect surgery, the wound is implanted with the gel of control group and then covered with Bio-Gide membrane; No. 3: after cranial defect surgery, the wound is implanted with the gel of experimental group 1 and then covered with Bio-Gide membrane; No. 4: after cranial bone defect surgery, the wound is implanted with the gel of experimental group 2 and then covered with Bio-Gide membrane; No. 5: after cranial bone defect surgery, the wound is implanted with the gel of experimental group 3 and then covered with Bio-Gide membrane; No. 6: after cranial bone defect surgery, the wound is implanted with the gel of experimental group 4 and then covered with Bio-Gide membrane; No. 7: positive control group: after cranial bone defect surgery, the wound is implanted with CoreBone 1000 bone powder and then covered with Bio-Gide membrane. The CoreBone 1000 used in the positive control group is a foreign commercial bone graft material in a granule form, which is produced by Israel CoreBone Company and certified by the European Union. The CoreBone 1000 is a natural bone graft material and has the advantages of high biocompatibility.

### DETAILED DESCRIPTION OF THE INVENTION

It is known that auto-crosslinked hyaluronic acid can be obtained by auto-crosslinking reaction in the presence of an activator, 2-chloro-1-methylpyridinium iodide (CMPI) (disclosed in US Patent No. 5,676,964), or in the presence of an acid (e.g., nitric acid or hydrochloric acid) at low temperature (e.g., Japanese Patent No. 4460663), but both of these existing technologies have their drawbacks.

The auto-crosslinking transesterification of hyaluronic acid using CMPI as an activator is a complex reaction involving a large amount of organic solvent, ion-exchange resin, and toxic reagent. The reaction invokes conversion of sodium hyaluronate to tetramonium hyaluronate, which is soluble in polar organic solvents, by means of a resin-filled column, in which the resin has to be treated with toxic TBAOH (tetrabutylammonium hydroxide). The resultant aqueous solution containing tetraammonium hyaluronate is freeze-dried, dissolved in an organic solvent such as dimethyl sulfoxide (DMSO), and then has CMPI added to carry out the auto-crosslinking reaction. During the reaction, triethylamine is added to neutralize the reaction-generated hydrochloric acid, which decreases pH of the solution. After the auto-crosslinking reaction, aqueous sodium chloride is added to the post-reaction mixture for removing the protective groups, and then a solvent (e.g., ethanol) is used to precipitate the auto-crosslinked hyaluronate, which is then dried to form auto-crosslinked hyaluronic acid. In the aforementioned process, when hyaluronic acid undergoes the substitution reaction of functional groups in the resin column, because of the high viscosity of the hyaluronic acid solution, the concentration of hyaluronic acid at the time of substitution is low (about 0.5 to 1.0 wt% hyaluronic acid). The low concentration of hyaluronic acid results in a large volume of the solution, which requires a large amount of solvents (e.g., ethanol) for precipitation. The tetraammonium salt of hyaluronate obtained by resin ion exchange has a limited solubility in organic solvent, which also requires a large amount of organic solvent during the esterification reaction, resulting in solvent recovery problems.

Another method for synthesizing auto-crosslinked hyaluronic acid is carried out at low temperature and in the presence of acid, without the use of organic solvents and toxic activators. The method is disclosed in Japanese Patent No. 4460663, and includes adjusting pH of a 1% hyaluronic acid solution to 2.0 or less with an acid solution (e.g., hydrochloric acid, nitric acid, and sulfuric acid), placing the hyaluronic acid solution at -20°C for 120 hours, and then washing the resultant product with a buffer solution of pH 7.0 and crushing the resultant product to obtain a hyaluronic acid gel. In the aforementioned washing step, soluble hyaluronic acid may be removed, thus the viscosity of the resultant hyaluronic acid gel is relatively low, which is unfavorable for practical pharmaceutical applications. Moreover, the gel obtained under such a low reaction concentration of hyaluronic acid cannot remain in gel state after steam treatment or sterilization, making it difficult to sustain sterility for application.

Additionally, when preparing a mixture containing hyaluronic acid at a high concentration for reaction, if mixing hyaluronic acid powder directly with a solution containing an acid (e.g., sulfuric acid, hydrochloric acid, or nitric acid), the mixture tends to form agglomerates containing hyaluronic acid powders inside. Such agglomerates are difficult to disperse or dissolve by stirring, which prevents hyaluronic acid from being efficiently mixed with the acid solution or requires a long stirring time, resulting in hydrolysis of hyaluronic acid. Accordingly, the subsequent auto-crosslinking reaction under low-temperature cannot be successfully carried out, and a large amount of hyaluronic acid not auto-crosslinked or auto-crosslinked to a low degree may be lost in subsequent washing. Thus, it is impossible to obtain auto-crosslinked hyaluronic acid particles or gels of stable nature through the aforementioned method.

In order to improve performance of auto-crosslinked hyaluronic acid gel as a biodegradable medical material, it is necessary to use hyaluronic acid having a high molecular weight and at a high concentration (e.g., 10 wt% or more) in reaction. However, since the colloid of hyaluronic acid with high molecular weight becomes very viscous with the increase in concentration, a long stirring time is required for the hyaluronic to be properly mixed with the acid, which also results in hydrolysis of the hyaluronic acid. For example, Comparative Example 1 of Japanese Patent No. 4383035 recites that 15 grams of nitric acid at a concentration of 1 N was placed in a mortar and 6 grams of sodium hyaluronate powder with a molecular weight of 2 million Dalton were mixed with the nitric acid (reaction concentration of the sodium hyaluronate is 28.6 wt%), and the mixture was kneaded thoroughly until uniformly mixed. Since the sodium hyaluronate immediately formed agglomerates with nitric acid, it was difficult to mix the mixture uniformly even after about 30 minutes of continuous kneading. In the continued kneading process, the molecular weight of the hyaluronic acid were reduced by nitric acid, thus forming a liquid-like mixture. After refrigeration at -20°C and freezing for a predetermined number of days, when the frozen product was thawed in a phosphate buffer, the sodium hyaluronate dissolved in the phosphate buffer and could not even form hyaluronic acid gel. The patent proposes an improved process of mixing hyaluronic acid and an acid solution, by first freezing the acid solution into fine granular or pulverized form, and then mixing it with hyaluronic acid powder at a low temperature of 0°C to -20°C, with a reaction concentration of the hyaluronic acid being 10 wt% or more. However, such process requires the acid solution to be frozen into fine granular or pulverized form, and mixing carried out continuously at low temperature. The process is complicated and unsuited to industrial production.

US Patent No. 9,216,193 discloses an auto-crosslinked hyaluronic acid composition comprising auto-crosslinked hyaluronic acid particles having an equilibrium swelling capacity of 3 to 10, a particle size of 10 to 100 µm, and a hyaluronic acid concentration of 3 to 8 wt%. The auto-crosslinked hyaluronic acid composition has a shear viscosity of less than 300 mPa·s measured at a shear rate of 50 sec⁻¹ and at 25±2°C, which can improve the problem of excessive thrust in the manufacture of high-concentration joint cavity injections. However, the patent follows the method used in Japanese Patent No. 4383035, in which the acid solution is first frozen into fine granular or pulverized form, and then mixed with hyaluronic acid powder at - 10°C. Therefore, the patent still has the disadvantages of mixing at low temperature and is complicated, which is unfavorable for industrial production. Meanwhile, the auto-crosslinked hyaluronic acid composition obtained by this method are in a granular form with a viscosity of only 300 mPa·s or less, which is not suitable for medical products that require high viscosity, such as wound dressings or post-operative anti-adhesive products.

Other techniques for synthesizing auto-crosslinked hyaluronic acid gels at low temperature and in the presence of acid include US 6,387,413, US 6,790,461, US 6,635,267, and US 7,014,860, wherein the gels obtained therefrom also show low viscosity after a washing step for removing acid and a neutralizing step. These patents also fail to demonstrate that after steam treatment or sterilization, the resultant gels can retain the desired condition and crosslinking property required for medical applications.

Unless otherwise defined, all technical and scientific terms used herein have the same meaning as commonly understood by one of ordinary skill in the art to which this disclosure pertains. In the case of conflict, the present document, including definitions, will control.

As used in this specification and the appended claims, the singular forms "a," "an" and "the" include plural references unless the content clearly dictates otherwise.

As used in this specification and the appended claims, the term "or" means "and/or" unless the content clearly dictates otherwise. In the case of multiple dependent claims, the term "or" is intended to refer back to more than one of the foregoing independent or dependent claims by way of substitution only.

The term "colloid of hyaluronic acid or a metal salt thereof" used herein refers to a colloid formed of solid powder or solid flocculent which swells by absorbing liquid solvent. Examples of the liquid solvent include, but are not limited to, distilled water, deionized water, or saline or buffer solutions.

The term "gel" used herein refers to a material having a storage modulus or modulus of elasticity (G') equal to or greater than a loss modulus or modulus of viscosity (G") thereof. The storage modulus or modulus of elasticity (G') and the loss modulus or modulus of viscosity (G") may be measured by a rheometer (e.g., AR2000ex rheometer, TA instruments) at a constant frequency. For example, when measured at 25°C and at each and every frequency between 0.1 to 10 Hz, the modulus of elasticity (G') of the "gel" may be equal to or greater than the modulus of viscosity (G"), and the loss tangent (tan δ, which equals to modulus of viscosity G"/modulus of elasticity G') is equal to or less than 1. In some embodiments of the present disclosure, a shear viscosity of the "gel" measured at 25°C and at a shear rate of 1 sec⁻¹ is at least 20 Pa·s or greater.

The present disclosure provides a method for synthesizing an auto-crosslinked hyaluronic acid gel at low temperature and in the presence of an acid, in which the auto-crosslinked hyaluronic acid gel can be steam-treated at high temperature. The resultant auto-crosslinked hyaluronic acid gel has a high viscosity and is sterile, which is suitable for medical applications such as anti-adhesion, bone regeneration and repair, subcutaneous fillers (or dermal fillers), joint disease treatment, etc. Specifically, the present disclosure provides a method for manufacturing an auto-crosslinked hyaluronic acid gel, comprising steps of:
(a) providing a colloid containing hyaluronic acid or a metal salt thereof;
(b) conducting an auto-crosslinking reaction of the colloid containing hyaluronic acid or a metal salt thereof in an acidic environment at about -10°C to about -30°C for about 7 days to about 42 days; and
(c) treating the product of step (b) by steam to obtain the auto-crosslinked hyaluronic acid gel.

In some embodiments of the present disclosure, the method is for manufacturing the auto-crosslinked hyaluronic acid gel exhibits at least one of:
a tan δ, at a concentration of about 1.5 wt% to about 7.5 wt% measured at 25°C and at each and every frequency between 0.1 Hz to 10.0 Hz, equal to or less than 1, a modulus of elasticity, at a concentration of about 1.5 wt% to about 7.5 wt% measured at 25°C and at 1.0 Hz, of about 30 Pa to about 8000 Pa, a modulus of viscosity, at a concentration of about 1.5 wt% to about 7.5 wt% measured at 25°C and at 1.0 Hz of about 20 Pa to 2000 Pa, and shear viscosity, at a concentration of about 1.5 wt% to about 7.5 wt% measured at 25°C and at a shear rate of 1 sec⁻¹, of about 20 Pa·s to about 900 Pa·s. In some embodiments of the present disclosure, the aforementioned concentration refers to the concentration of hyaluronic acid of a salt thereof in the auto-crosslinked hyaluronic acid gel.

In some embodiments of the present disclosure, before the auto-crosslinking reaction, the hyaluronic acid or a metal salt thereof (which may be in a powder or granular form) is soaked in or mixed with an aqueous solvent to absorb the same and thus form a colloid containing hyaluronic acid or a metal salt thereof approaching solidity. In some embodiments of the present disclosure, the aqueous solvent is distilled water or deionized water. While not intending to be bound by any theory, it is believed that since the powder of hyaluronic acid or a metal salt thereof absorbs the aqueous solvent and swells uniformly to form the colloid, the acid can be readily and adequately mixed with the colloid containing hyaluronic acid or a metal salt thereof by stirring. In some embodiments of the present disclosure, the colloid containing hyaluronic acid or a metal salt thereof may be mixed with the acid at room temperature without forming agglomerates containing hyaluronic acid powder, which can overcome the drawbacks of agglomeration and mixing at low temperature in prior art. The mixture can be maintained at low temperature for a sufficient period of time to auto-crosslink, and then optionally homogenized, washed, and neutralized. After removal of the aqueous solvent, auto-crosslinked hyaluronic acid particles may be obtained. The auto-crosslinked hyaluronic acid particles may then be treated by steam at high temperature to form a sterile, high viscosity auto-crosslinked hyaluronic acid gel. The method of the present disclosure improves the low viscosity of the crosslinked hyaluronic acid produced by conventional low-temperature synthesis, and can be used in medical applications such as post-operative anti-adhesion, bone regeneration, and repair of defects, dermal fillers, and arthropathy treatment, etc.

In some embodiments of the present disclosure, the auto-crosslinking of hyaluronic acid is an esterification reaction between inter- or intramolecular hydroxyl groups (-OH) and carboxyl groups (-COOH) of hyaluronic acid. The method of the present disclosure uses no crosslinking agents, and is merely achieved through the recited auto-crosslinking reaction.

The average molecular weight of the hyaluronic acid or a metal salt thereof according to the present disclosure is not particularly limited, provided that the hyaluronic acid or a metal salt thereof can be used for the formation of colloids and gels. In some embodiments of the present disclosure, the average molecular weight of the hyaluronic acid or a metal salt thereof is about 766,000 or greater (intrinsic viscosity η=1.4 m³/kg; average molecular weight, calculated by Laurent's formula (molecular weight={[η]×10⁵/3.6}^{1/0.78}), of 766,000). In some embodiments of the present disclosure, the average molecular weight of the hyaluronic acid or a metal salt thereof is about 983,000 to about 1,121,000 (with an intrinsic viscosity of about 1.7 m³/kg to about 2.0 m³/kg). The intrinsic viscosity of the hyaluronic acid or a metal salt thereof may be measured according to pharmacopoeia, such as that described in European Pharmacopoeia 9.0.

In some embodiments of the present disclosure, step (a) comprises providing the colloid containing hyaluronic acid or a metal salt thereof in a granule form, a stripe form, or a string form. For example, the colloid containing hyaluronic acid or a metal salt thereof can be crushed into small pieces (e.g., granules, stripes or strings) before the auto-crosslinking reaction in step (b). The crushing method is not limited to the present disclosure. For example, the colloid containing hyaluronic acid or a metal salt thereof may be crushed, sieved, or chopped through a syringe, a sieve, or by metal, such as a blade.

In some embodiments of the present disclosure, the reaction temperature of step (b) is about -10°C to about -30°C, such as about -10°C to about -20°C.

In some embodiments of the present disclosure, the acidic environment can be provided with any kind of acid at any concentration, provided that the hyaluronic acid or a metal salt thereof can sufficiently auto-crosslink in the acidic environment to form the auto-crosslinked hyaluronic acid gel. In some embodiments of the present disclosure, the acidic environment is provided by an acid at a concentration of about 0.5 N to about 1.5 N, and the acid is selected from the group consisting of hydrochloric acid, nitric acid, and sulfuric acid.

In some embodiments of the present disclosure, step (b) comprises mixing the colloid containing hyaluronic acid or a metal salt thereof with an acid at room temperature to form a mixture, and letting the mixture stand at about -10°C to about -30°C for about 7 to 42 days. The acid may be prepared as a solution (e.g., aqueous solution) of any concentration before mixing, provided that the concentration of the acid in the mixture can create a proper environment for the auto-crosslinking reaction of hyaluronic acid, such as about 0.5 N to 1.5 N. In some embodiments of the present disclosure, the mixture is prepared by mixing the colloid containing hyaluronic acid or a metal salt thereof with an adequate amount of a 14 N nitric acid solution, a 12 N hydrochloric acid solution, or a 12 N sulfuric acid solution. Alternatively, in some embodiments of the present disclosure, the mixture is prepared by mixing the colloid containing hyaluronic acid or a metal salt thereof with an adequate amount of a hydrochloric acid solution, a nitric acid solution, or a sulfuric acid solution at a concentration of about 6 N to about 12 N.

In some embodiments of the present disclosure, the hyaluronic acid or a metal salt thereof reaches a predetermined concentration in the mixture. In some embodiments of the present disclosure, a concentration of hyaluronic acid or a metal salt thereof in the mixture is about 10 wt% to about 30 wt%, such as about 10 wt% to about 20 wt%, or about 10 wt% to about 14.5 wt%.

The manner of mixing the colloid containing hyaluronic acid or a metal salt thereof with the acid is not limited to the present disclosure, provided that homogeneous mixing can be achieved. The mixing can be carried out by manpower or machine, for example, but is not limited to, hand-held mixing stick, or machine such as a kneader, stirrer, blender, or planetary centrifugal mixer.

In some embodiments of the present disclosure, the acid concentration in the mixture associates or correlates with the reaction temperature of the auto-crosslinking reaction and the concentration of the hyaluronic acid or a salt thereof. In some embodiments of the present disclosure, the acid concentration in the mixture may be about 0.58 N to about 1.0 N for a reaction temperature of about -10°C. The acid concentration in the mixture may be about 0.58 N to about 1.5 N for a reaction temperature of about -20°C. The acid concentration in the mixture may be about 1.0 N to about 1.5 N for a reaction temperature of about -30°C. In some embodiments of the present disclosure, the acid concentration in the mixture may be about 0.58 N to about 1.0 N for a reaction temperature of about -10°C with the concentration of the hyaluronic acid or a salt thereof in the mixture being about 10 wt %.

In some embodiments of the present disclosure, the reaction time of the auto-crosslinking reaction may be at least 7 days or 14 days, which varies based on the reaction temperature and the concentration of the hyaluronic acid or a salt thereof in the mixture.

In some embodiments of the present disclosure, with the concentration of the hyaluronic acid or a salt thereof in the mixture being about 14.5 wt %, and the reaction temperature of about -10°C, reaction time may be about 14 days to about 42 days, such as about 21 days to about 28 days.

In some embodiments of the present disclosure, with the concentration of the hyaluronic acid or a salt thereof in the mixture being about 14.5 wt %, and the reaction temperature of about -20°C, the reaction time may be about 7 days to about 35 days, such as about 14 days to about 28 days.

In some embodiments of the present disclosure, with the concentration of the hyaluronic acid or a salt thereof in the mixture being about 14.5 wt %, and the reaction temperature of about -30°C, the reaction time may be about 14 days to about 35 days, such as about 14 days to about 28 days.

In some embodiments of the present disclosure, with the concentration of the hyaluronic acid or a salt thereof in the mixture being about 10 wt %, and the reaction temperature of about -10°C, the reaction time may be about 7 days to about 28 days, such as about 14 days to about 28 days.

In some embodiments of the present disclosure, with the concentration of the hyaluronic acid or a salt thereof in the mixture being about 10 wt %, and the reaction temperature of about -20°C, the reaction time may be about 7 days to about 28 days, such as about 14 days to about 28 days.

In some embodiments of the present disclosure, with the concentration of the hyaluronic acid or a salt thereof in the mixture being about 10 wt %, and the reaction temperature of about -30°C, the reaction time may be about 14 days to about 35 days, such as about 14 days to about 28 days.

In some embodiments of the present disclosure, the method comprises conducting the auto-crosslinking reaction only once, continuously at about -10°C to about -30°C. That is, after mixing, the mixture is kept at about -10°C to about -30°C throughout the entire reaction time of the auto-crosslinking reaction. In some embodiments of the present disclosure, the reaction temperature may be substantially constant throughout the entire auto-crosslinking reaction.

In some embodiments of the present disclosure, before step (c), the method further comprises homogenizing and/or washing the product of step (b). In some embodiments of the present disclosure, before (c), the method further comprises homogenizing and washing the product of step (b), thus forming auto-crosslinked hyaluronic acid particles.

In some embodiments of the present disclosure, the product of step (b) is homogenized prior to the washing step. In some embodiments of the present disclosure, the homogenizing step of the present disclosure means to reduce the size of the produce of step (b), thus making the produce of step (b) dispersed evenly in a liquid, such as the liquid used in the washing step. The homogenizing step of the present disclosure may include crushing, and may be achieved by a homogenizer or through a sieve. In some embodiments of the present disclosure, the product of step (b) is homogenized by a homogenizer.

The washing step may use water, distilled water, deionized water, saline, or a buffering solution. The washing step removes acidic components such as nitric acid, hydrochloric acid, or sulfuric acid from the product of step (b), and neutralizes the resultant product to neutral or near neutral pH.

While not intending to be bound by any theory, it is believed that the pH of the auto-crosslinked hyaluronic acid particles before steam treatment may affect the rheological properties of the auto-crosslinked hyaluronic acid gel after steam treatment. In some embodiments of the present disclosure, the pH of the auto-crosslinked hyaluronic acid particles is adjusted with a buffer solution, a weak acid, and/or a weak base before the steam treatment in step (c). Since the suspended aqueous solvent is removed by sieving or filtration in the washing step, the pH of the auto-crosslinked hyaluronic acid particles cannot be easily measured, therefore the pH of the waste solvent of washing is measured as a substitute. The pH prior to steam treatment can be adjusted to about pH 7.0 to about pH 6.0, such as about pH 6.3 to about 6.8.

The manner of washing and adjusting the pH of the product of step (b) and/or the auto-crosslinked hyaluronic acid particles is not limited to the present disclosure, provided that a predetermined pH of the auto-crosslinked hyaluronic acid particles can be reached. For example, this can be done alone with a buffer solution of the target pH or with the assistance of titration with an acid or basic solution at a low concentration. Since the auto-crosslinked hyaluronic acid particles tend to hydrolyze rapidly in basic buffer solutions, it is important to protect them from hydrolysis due to the high pH of the washing process. In some embodiments of the present disclosure, the product of step (b) is washed with a neutral buffer solution to neutral or near neutral pH, and the suspended aqueous solvent is removed. Then, the auto-crosslinked hyaluronic acid particles is washed with a buffer solution of the target pH at least once, and then adjusted to the target pH by titration with an acid or basic solution at a low concentration (e.g., a hydrochloric acid or sodium hydroxide solution at 0.1 N). For example, after the low-temperature reaction, the reaction product can be first situated at room temperature, and then added to a certain amount of 100 mM phosphate buffer solution of pH 7.0 and homogenized by crushing. After removing the buffer solution by filtration, the product is washed with fresh 100 mM phosphate buffer solution of pH 7.0 several times until the solution has a pH of about 7.0. Then, the product is washed with buffer solution of a target pH (e.g., pH 7.0, pH 6.8, pH 6.5, pH 6.3, and pH 6.0) at least once, and then titrated to the target pH with a weak acid or base solution. Such process may minimize hydrolysis of the reaction product during washing and/or pH adjustment.

The components and concentration of the buffer solution used are not limited to the present disclosure, provided they are suitable for medical uses of humans. In some embodiments of the present disclosure, the buffer solution may be a phosphate buffer solution at a concentration of about 1 mM to about 1000 mM, of about 10 mM to about 300 mM, or of about 10 mM to about 100 mM. The pH of the buffer solution may be about pH 6.0 to about 7.0. For example, a phosphate buffer solution containing 100 mM sodium phosphate or potassium phosphate solution and having a pH of about 6.0 to about 7.0 may be used.

After the homogenizing, washing and pH adjusting steps, the auto-crosslinked hyaluronic acid particles automatically separate from the aqueous phase into a single layer simply by standing for a period of time. Those auto-crosslinked hyaluronic acid gels, which do not automatically separate from the aqueous phase or which form suspension with the aqueous phase, cannot maintain their crosslinked state after steam treatment due to their weak crosslinked structure.

The method of the present disclosure comprises a step (c) of treating the product of step (b) by steam, such at about 110°C to about 130°C for about 10 minutes to about 60 minutes, to obtain the auto-crosslinked hyaluronic acid gel.

According to the present disclosure, the structure of the resultant auto-crosslinked hyaluronic acid gel can be altered by adjusting the temperature and time of the steam treatment, which results in alteration of the viscoelastic properties, such as modulus of elasticity (G'), viscosity modulus (G") and shear viscosity.

The manner of steam treatment is not particularly limited to the present disclosure, provided that it is sufficient to convert the auto-crosslinked hyaluronic acid particles into sterile, high viscosity auto-crosslinked hyaluronic acid gel. In some embodiments of the present disclosure, the pH-adjusted auto-crosslinked hyaluronic acid particles are filled into glass or plastic containers (e.g., glass or plastic syringes), and then treated by high-temperature steam. In some embodiments of the present disclosure, the temperature of the steam is about 110°C to about 130°C, about 115°C to about 125°C, or about 120°C to about 121°C. The time of steam treatment is set based on the steam temperature and the required viscoelastic properties of the gel, provided that the auto-crosslinked hyaluronic acid can be converted from a granular form to a gel form which is sterile while maintaining its crosslinked state. For example, for steam treatment at 121°C, the treatment time may be about 10 minutes to about 60 minutes, about 15 minutes to about 60 minutes, or about 15 minutes to about 35 minutes. Different treatment times may result in different viscoelastic properties of the auto-crosslinked hyaluronic acid gel. The sterility of the auto-crosslinked hyaluronic acid gel of the present disclosure may be verified by the sterility test method of pharmacopoeia (e.g., European Pharmacopoeia version 9.0).

The volume of the syringe or container used in the steam treatment of the present disclosure may be about 0.5 mL to about 50 mL, about 1 mL to about 10 mL, or about 3 mL to about 10 mL.

The literature (K. EDSMAN et al., "Gel properties of hyaluronic acid dermal fillers", Dermatol Surg. vol. 38, p1170- 1179 (2012)) states that in the case of linear hyaluronic acid, when measured at low frequencies, the modulus of elasticity (G') thereof is lower than the modulus of viscosity (G") thereof; while measure at high frequencies, the modulus of elasticity (G') thereof is higher than the modulus of viscosity (G") thereof. In contrast, in the case of crosslinked hyaluronic acid, due to the crosslinked network structure, the modulus of elasticity (G') thereof is higher than the modulus of viscosity (G") thereof regardless of the frequency of measurement. Such properties of hyaluronic acid gel can be used to determine whether it is linear or crosslinked. Therefore, the auto-crosslinked hyaluronic acid gel of the present disclosure refers to a hyaluronic acid gel after steam treatment, in which the modulus of elasticity (G') thereof measured at 25°C and at each and every frequency between 0.1 Hz to 10.0 Hz is equal to or greater than the modulus of viscosity (G") thereof, and the tan δ is less than or equal to 1. In addition, the shear viscosity of the auto-crosslinked hyaluronic acid gel of the present disclosure measured at a shear rate of 1 sec⁻¹ and at 25°C is at least 20 Pa·s or greater. On the other hand, if the hyaluronic acid gel has the modulus of elasticity (G') lower than the modulus of viscosity (G") at any frequency between 0.1 Hz to 10.0 Hz, it is determined to be linear.

In some embodiments of the present disclosure, the viscoelasticity of the auto-crosslinked hyaluronic acid gel may be measured by a rheometer (TA, AR2000ex) with 20 mm metal plate, at 25°C and at 1.0 Hz. The modulus of elasticity (G') is about 30 Pa to about 8000 Pa, about 200 Pa to about 1000 Pa, or about 300 Pa to about 800 Pa. The modulus of viscosity (G") is about 20 Pa to about 2000 Pa, about 100 Pa to about 600 Pa, or about 150 Pa to about 500 Pa. The modulus of elasticity and the modulus of viscosity of the auto-crosslinked hyaluronic acid gel decrease with the increase of the time of the steam treatment.

In some embodiments of the present disclosure, the shear viscosity of the auto-crosslinked hyaluronic acid gel measured at a shear rate of 1 sec⁻¹ and at 25°C is about 20 Pa·s to about 900 Pa·s, about 100 Pa·s to about 800 Pa·s, or about 250 Pa·s to about 700 Pa·s. In some further embodiments, the shear viscosity of the auto-crosslinked hyaluronic acid gel measured at 25°C and at a shear rate of 1 sec⁻¹ is about 38.04 Pa·s to about 896.91 Pa·s, or about 55.69 Pa·s to about 896.91 Pa·s. In some embodiments of the present disclosure, a shear viscosity of the auto-crosslinked hyaluronic acid gel measured at 25°C and at a shear rate of 50 sec⁻¹ is about 0.31 Pa·s or greater, such as about 0.35 Pa·s or greater, about 0.4 Pa·s or greater, about 0.5 Pa·s or greater, about 0.6 Pa·s or greater, about 0.8 Pa·s or greater, or about 1 Pa·s or greater. In some further embodiments, the shear viscosity of the auto-crosslinked hyaluronic acid gel measured at 25°C and at a shear rate of 50 sec⁻¹ is about 1.97 Pa·s to about 35.66 Pa·s, or about 2.18 Pa·s to about 35.66 Pa·s.

In some embodiments of the present disclosure, the equilibrium swelling capacity of the auto-crosslinked hyaluronic acid particles is determined as follows. 1 g of a sample of auto-crosslinked hyaluronic acid particles, which has been homogenized, washed and neutralized to about pH 7.0, is soaked in a pH 6.0 buffer solution containing 0.9 wt % sodium chloride and 10 mM sodium phosphate at 5°C for 24 hours to swell to an equilibrium state, and then dried at 25°C to a constant weight. Since the buffer solution-swollen auto-crosslinked hyaluronic acid particles are in a granule form, the density thereof cannot be measured. Hence, the equilibrium swelling capacity in the present disclosure is determined by the following formula.

Equilibrium swelling capacity = (wet weight of the buffer solution-swollen auto-crosslinked hyaluronic acid particles / dry weight of the buffer solution-swollen auto-crosslinked hyaluronic acid particles)

In some embodiments of the present disclosure, the equilibrium swelling capacity of the auto-crosslinked hyaluronic acid particles is greater than 10, such as about 12 to about 30, about 12 to about 20, about 13 to about 18, or about 13 to about 16.

Typically, hyaluronic acid with more efficient crosslinking produces a tighter crosslinking network, thus the colloid or particles thereof may be stiffer, resulting in a lower swelling ratio (Mohammed Al-Sibani et al., "Study of the effect of mixing approach on cross-linking efficiency of hyaluronic acid-based hydrogel cross-linked with 1,4-butanediol diglycidyl ether", European Journal of Pharmaceutical Sciences, Vol. 91, pp. 131-137(2016)). Other literature also states that a higher crosslinking density results in a lower swelling ratio (Noel L. Davison et al., "Degradation of Biomaterials", Tissue Engineering (Second Edition), chapter 6, Elsevier Inc., pp. 197(2014)).

As the reaction time increases, the hyaluronic acid concentration of the resultant auto-crosslinked hyaluronic acid particles increases, the recovery rate increases, and the equilibrium swelling capacity decreases. As the reaction temperature decreases, the hyaluronic acid concentration of the auto-crosslinked hyaluronic acid particles decreases, the recovery rate decreases, and the equilibrium swelling capacity increases. In some embodiments of the present disclosure, the recovery rate of hyaluronic acid is between about 70% and 100%.

In addition, after being adjusted to a pH between 6.0 to 7.0, if directly dried to a constant weight at 25°C, the equilibrium swelling capacity is substantially the same for auto-crosslinked hyaluronic acid particles of different pHs. The equilibrium swelling capacity of auto-crosslinked hyaluronic acid particles is independent of pH at least in the range of 6.0 to 7.0, which is nearly the same as the aforementioned equilibrium swelling capacity of the auto-crosslinked hyaluronic acid particles swollen to an equilibrium state in a pH 6.0 buffer solution containing 0.9 wt% sodium chloride and 10 mM sodium phosphate at 5°C for 24 hours.

The average particle size of the auto-crosslinked hyaluronic acid particles of the present disclosure before steam treatment is not particularly limited, and can be obtained with different homogenizers, at different rotational speeds, and for different homogenization times. In some embodiment of the present disclosure, an average particle size of the auto-crosslinked hyaluronic acid particles is about 200 µm to about 3000 µm, about 600 µm to about 2500 µm, or about 1500 µm to about 2200 µm. The average particle size is determined using saline as a suspending solvent, and measured with a particle size measuring instrument (PARTICA LA-960, HORIBA) at 25°C.

In some embodiments of the present disclosure, the pH of the auto-crosslinked hyaluronic acid gel after steam treatment is about 4.5 to about 6.5, about 5.0 to about 6.0, or about 5.1 to about 5.8.

The hyaluronic acid concentration of the auto-crosslinked hyaluronic acid gel of the present disclosure is determined by the Carbazole Assay of pharmacopoeia (e.g., European Pharmacopoeia version 9.0). The hyaluronic acid concentration of the auto-crosslinked hyaluronic acid gel of the present disclosure increases with the increase of the reaction time and the reaction temperature, which concentration is about 1.5 wt% to about 7.5 wt%, about 2.0 wt% to about 6.0 wt%, about 3.0 wt% to about 6.0 wt%, or about 4.0 wt% to about 6.0 wt%. In the present disclosure, the hyaluronic acid concentration of the auto-crosslinked hyaluronic acid gel after steam treatment will be the same as the hyaluronic acid concentration of the auto-crosslinked hyaluronic acid particles before steam treatment.

The auto-crosslinked hyaluronic acid gel of the present disclosure may also be obtained by mixing a linear hyaluronic acid solution or an aqueous phase (e.g., distilled water, saline, or buffered solution) with the auto-crosslinked hyaluronic acid particles of the present disclosure, and then treating the resultant mixture with steam. Provided that the product is determined to meet the definition of auto-crosslinked hyaluronic acid gel as defined in the present disclosure, it is within the scope of the present disclosure.

In some embodiments of the present disclosure, the osmotic pressure of the auto-crosslinked hyaluronic acid gel after steam treatment can be controlled by the osmotic pressure of an aqueous solution or a buffer solution used in the washing step and/or the pH adjusting step, thus is not particularly limited, provided that it is suitable for medical use on humans. The osmotic pressure of the auto-crosslinked hyaluronic acid gel of the present disclosure after steam treatment can be about 10 to about 350 mOsm/kg, about 30 to about 200 mOsm/kg, or about 50 to about 150 mOsm/kg.

The auto-crosslinked hyaluronic acid gel of the present disclosure can be used for treating bone regeneration and defects. The experimental method involves cranial bone defects in rats (Patrick P Spicer et al., "Evaluation of bone regeneration using the rat critical size calvarial defect", Nature Protocol, vol. 7, pp. 1918-1929(2012)), in which the wound is covered by the auto-crosslinked hyaluronic acid gel. The rats are sacrificed at three months after the surgery, and the bone is removed and analyzed by microcomputed tomography. The result is compared with a commercial product group, a linear hyaluronic acid group, a blank control group (with no substance added after the surgery), and a positive control group. The method for manufacturing an auto-crosslinked hyaluronic acid gel of the present disclosure is simple and free of using toxic organic solvents or crosslinking agents, and the resultant auto-crosslinked hyaluronic acid gel shows favorable bone repair and regeneration effects in animal experiments.

The present disclosure provides a modified manufacturing method for synthesizing auto-crosslinked hyaluronic acid particles under low temperature. After adjustment to a predetermined pH, the auto-crosslinked hyaluronic acid particles can be treated by steam to form a sterile, high-viscosity auto-crosslinked hyaluronic acid gel, which can be used for medical applications such as bone regeneration and defect repairment, post-operative anti-adhesion, subcutaneous fillers, and arthropathy treatment.

The following Examples are given for the purpose of illustration only and are not intended to limit the scope of the present invention.

### Example 1: reaction condition: hyaluronic acid (HA) concentration 14.5 wt %, 0.58 N HCl, reaction temperature -10°C

12 grams of hyaluronic acid with a water content of 3.65 wt%, an intrinsic viscosity of 1.7 m³/kg and an average molecular weight of 983,000 were add into 59.57 grams of distilled water, and mixed in a mixer (SPAR FOOD MACHINERYMFG. CO., LTD., Model SP-502A) at 253 rpm for 5 minutes to form water-swollen colloid containing hyaluronic acid. The colloid was extruded through a metal sieve having an aperture size of about 0.2 cm into stripes, and then mixed with 8.43 g of 6 N hydrochloric acid (density 1.1 g/cm³) in the mixer at the same speed for 5 minutes to form a colloid containing hyaluronic acid and hydrochloric acid in a homogeneous manner. The resultant colloid was placed in a serum bottle and kept in refrigerator for 14 days at low temperature as shown in Table 1 for auto-crosslinking reaction. After the reaction, the product was thawed at room temperature, then added into 600 mL of 100 mM phosphate buffer solution of pH 7.00 and homogenized by a homogenizer (IKA, model T-25) at 10,000 rpm for 1 minute. Then, the buffer solution was removed by sieving, and the product was then added into another 600 mL of fresh 100 mM phosphate buffer solution of pH 7.0 and homogenized at 6,000 rpm for 5 minutes by a high-speed emulsifying homogenizer (Gordon, Model HM25). The buffer solution was removed by sieving, and the product was washed with 3000 mL fresh buffer solution several times until the buffer solution had a stable pH of 7.00±0.10. After filtration for removing the buffer solution, the resultant product was weighed and divided into five equal portions, and then separately washed by 100 mM phosphate buffer solution at the target pH (pH 7.00, pH 6.80, pH 6.50, pH 6.30, and pH 6.00) with 10 times the weight of the product, and then titrated with aqueous solution of 0.1 N hydrochloric acid or sodium hydroxide until the solution reached a stabilized pH equal to the target pH (pH 7.00, pH 6.80, pH 6.50, pH 6.30, and pH 6.00). The resultant hyaluronic acid particles were filtered with a sieve to remove the solution, and then filled into a 1 mL syringe and treated with steam at 121°C for different fixed times to convert the auto-crosslinked hyaluronic acid particles into an auto-crosslinked hyaluronic acid gel. For experiments with the reaction time of 21, 28, 35, and 42 days, the steps of the method and the reaction conditions were the same as those described, except for the low-temperature reaction time set. The resultant auto-crosslinked hyaluronic acid gel was incubated for 14 days for a microbial sterility test, and confirmed to be free of microbial growth (negative).

**Table 1: The reaction conditions and the properties of the auto-crosslinked hyaluronic acid gels which were auto-crosslinked at -10°C in the presence of 0.58 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 15 | 4496.51 | 769.45 | 0.17 | 298.48 | 0.89 | 6.17 |
| | | 25 | 335.20 | 170.29 | 0.51 | 239.64 | 16.57 | 6.19 |
| | | 35 | 41.13 | 51.54 | 1.25 | 48.49 | 5.79 | 6.15 |
| | 6.8 | 15 | 6451.02 | 1145.19 | 0.18 | 202.74 | 0.49 | 5.86 |
| | | 25 | 1790.61 | 427.43 | 0.24 | 396.62 | 2.75 | 5.82 |
| | | 35 | 573.38 | 275.79 | 0.48 | 361.56 | 7.45 | 5.83 |
| | 6.5 | 15 | 6155.41 | 1416.70 | 0.23 | 173.41 | 0.35 | 5.57 |
| | | 25 | 1421.77 | 352.38 | 0.25 | 425.96 | 3.93 | 5.52 |
| | | 35 | 290.91 | 248.35 | 0.85 | 225.35 | 16.56 | 5.55 |
| | 6.3 | 15 | 5571.40 | 879.75 | 0.16 | 256.02 | 0.58 | 5.13 |
| | | 25 | 779.63 | 331.35 | 0.43 | 540.40 | 7.31 | 5.10 |
| | | 35 | 256.73 | 140.24 | 0.55 | 200.35 | 18.54 | 5.14 |
| | 6.0 | 15 | 542.30 | 221.30 | 0.41 | 208.86 | 9.67 | 4.92 |
| | | 25 | 16.51 | 29.80 | 1.80 | 12.55 | 3.61 | 4.93 |
| | | 35 | 0.80 | 2.45 | 3.06 | 0.41 | 0.47 | 4.88 |
| 21 | 7.0 | 15 | 4749.22 | 757.86 | 0.16 | 281.80 | 0.70 | 6.28 |
| | | 25 | 3063.80 | 579.85 | 0.19 | 334.50 | 1.89 | 6.25 |
| | | 35 | 424.99 | 142.16 | 0.33 | 173.98 | 19.66 | 6.26 |
| | 6.8 | 15 | 7231.22 | 1495.21 | 0.21 | 260.81 | 1.17 | 5.92 |
| | | 25 | 5643.52 | 1076.81 | 0.19 | 255.99 | 0.57 | 5.89 |
| | | 35 | 2897.81 | 642.57 | 0.22 | 414.18 | 2.67 | 5.88 |
| | 6.5 | 15 | 7183.26 | 1457.06 | 0.20 | 138.16 | 0.49 | 5.54 |
| | | 25 | 3715.20 | 644.73 | 0.17 | 367.13 | 0.63 | 5.51 |
| | | 35 | 1680.48 | 628.73 | 0.37 | 180.88 | 5.63 | 5.50 |
| | 6.3 | 15 | 6017.85 | 937.29 | 0.16 | 326.90 | 0.74 | 5.21 |
| | | 25 | 3238.92 | 704.25 | 0.22 | 478.01 | 1.19 | 5.12 |
| | | 35 | 1077.12 | 341.66 | 0.32 | 293.92 | 6.71 | 5.08 |
| | 6.0 | 15 | 689.81 | 191.95 | 0.28 | 221.65 | 7.66 | 4.96 |
| | | 25 | 280.79 | 153.16 | 0.55 | 204.49 | 19.86 | 4.93 |
| | | 35 | 1.83 | 7.84 | 4.28 | 1.61 | 1.22 | 4.76 |
| 28 | 7.0 | 35 | 756.82 | 296.61 | 0.39 | 305.92 | 5.68 | 6.38 |
| | | 45 | 137.03 | 79.39 | 0.58 | 139.92 | 29.11 | 6.25 |
| | 6.8 | 35 | 3377.44 | 610.79 | 0.18 | 349.51 | 0.66 | 5.93 |
| | | 45 | 2905.56 | 591.05 | 0.20 | 488.06 | 2.58 | 5.84 |
| | 6.5 | 35 | 2368.80 | 541.66 | 0.23 | 430.62 | 3.55 | 5.41 |
| | | 45 | 2130.40 | 581.93 | 0.27 | 581.42 | 5.82 | 5.26 |
| | 6.3 | 35 | 2092.18 | 437.02 | 0.21 | 347.38 | 6.52 | 5.12 |
| | | 45 | 1024.36 | 262.27 | 0.26 | 251.29 | 8.17 | 5.06 |
| | 6.0 | 35 | 38.27 | 57.44 | 1.50 | 36.46 | 7.59 | 4.87 |
| | | 45 | 0.93 | 5.01 | 5.39 | 0.87 | 0.81 | 4.76 |
| 35 | 7.0 | 45 | 606.76 | 233.48 | 0.38 | 214.09 | 11.21 | 6.22 |
| | | 55 | 9.27 | 26.45 | 2.85 | 7.39 | 4.13 | 6.20 |
| | 6.8 | 45 | 5514.16 | 899.47 | 0.16 | 371.59 | 0.58 | 5.85 |
| | | 55 | 628.39 | 331.19 | 0.53 | 470.41 | 13.13 | 5.83 |
| | 6.5 | 45 | 3566.83 | 776.05 | 0.22 | 419.89 | 1.40 | 5.46 |
| | | 55 | 585.90 | 347.86 | 0.59 | 878.23 | 26.02 | 5.42 |
| | 6.3 | 45 | 2174.89 | 447.00 | 0.21 | 308.99 | 2.30 | 5.17 |
| | | 55 | 115.42 | 96.38 | 0.84 | 290.90 | 13.42 | 5.15 |
| | 6.0 | 45 | 16.05 | 30.90 | 1.93 | 14.05 | 4.33 | 4.96 |
| | | 55 | 0.94 | 3.86 | 4.11 | 0.68 | 0.83 | 4.93 |
| 42 | 7.0 | 45 | 1248.02 | 285.20 | 0.23 | 259.46 | 5.65 | 6.32 |
| | | 55 | 32.49 | 51.05 | 1.57 | 36.56 | 8.19 | 6.21 |
| | 6.8 | 45 | 7350.55 | 1193.88 | 0.16 | 217.91 | 0.52 | 5.83 |
| | | 55 | 1058.53 | 390.42 | 0.37 | 370.00 | 6.03 | 5.75 |
| | 6.5 | 45 | 6365.97 | 1138.66 | 0.18 | 495.95 | 0.93 | 5.47 |
| | | 55 | 883.13 | 442.08 | 0.50 | 553.64 | 4.47 | 5.32 |
| | 6.3 | 45 | 4211.30 | 703.68 | 0.17 | 293.12 | 0.75 | 5.18 |
| | | 55 | 193.51 | 125.21 | 0.65 | 251.55 | 31.42 | 5.05 |
| | 6.0 | 45 | 121.81 | 92.01 | 0.76 | 274.04 | 25.89 | 4.92 |
| | | 55 | 1.81 | 8.89 | 4.91 | 1.66 | 1.31 | 4.76 |

### Example 2: reaction condition: hyaluronic acid concentration 14.5 wt %, 1.0 N HCl, reaction temperature -10°C

The steps of the method and the reaction conditions of Example 2 were the same as those of Example 1, except that the amount of distilled water was 53.47 g, the amount of 6 N HCl was 14.53 g, and the reaction time was 14 days, 21 days, and 28 days.

**Table 2: The reaction conditions and the properties of the auto-crosslinked hyaluronic acid gels which were auto-crosslinked at -10°C in the presence of 1.0 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 33.19 | 37.82 | 1.14 | 35.93 | 3.90 | 6.43 |
| | | 15 | 29.66 | 37.21 | 1.25 | 23.91 | 3.83 | 6.41 |
| | | 20 | 28.34 | 36.75 | 1.30 | 21.02 | 3.56 | 6.38 |
| | 6.8 | 10 | 143.73 | 66.51 | 0.46 | 98.75 | 8.02 | 6.21 |
| | | 15 | 116.36 | 58.31 | 0.50 | 83.21 | 6.12 | 6.22 |
| | | 20 | 76.20 | 54.22 | 0.71 | 135.17 | 8.98 | 6.17 |
| | 6.5 | 10 | 786.64 | 149.97 | 0.19 | 153.47 | 3.98 | 5.93 |
| | | 15 | 267.74 | 83.78 | 0.31 | 89.02 | 5.89 | 5.90 |
| | | 20 | 123.66 | 57.00 | 0.46 | 81.13 | 8.00 | 5.87 |
| | 6.3 | 10 | 1702.04 | 254.86 | 0.15 | 148.30 | 1.53 | 5.74 |
| | | 15 | 1348.42 | 263.11 | 0.20 | 131.29 | 1.78 | 5.74 |
| | | 20 | 851.35 | 177.61 | 0.21 | 135.44 | 3.66 | 5.72 |
| | 6.0 | 10 | 958.45 | 171.96 | 0.18 | 111.76 | 2.36 | 5.48 |
| | | 15 | 868.36 | 133.28 | 0.15 | 103.02 | 1.97 | 5.45 |
| | | 20 | 342.76 | 83.88 | 0.24 | 83.62 | 5.40 | 5.41 |
| 21 | 7.0 | 15 | 231.77 | 112.13 | 0.48 | 162.71 | 6.58 | 6.25 |
| | | 20 | 40.70 | 47.50 | 1.17 | 46.77 | 4.82 | 6.21 |
| | | 25 | 35.12 | 45.45 | 1.29 | 29.18 | 4.58 | 6.20 |
| | 6.8 | 15 | 1307.03 | 310.41 | 0.24 | 139.77 | 1.61 | 6.07 |
| | | 20 | 182.99 | 97.14 | 0.53 | 168.95 | 7.41 | 6.05 |
| | | 25 | 116.04 | 70.55 | 0.61 | 100.55 | 6.60 | 6.02 |
| | 6.5 | 15 | 2200.22 | 476.95 | 0.22 | 97.87 | 0.64 | 5.78 |
| | | 20 | 1100.96 | 239.77 | 0.22 | 152.06 | 2.86 | 5.73 |
| | | 25 | 786.66 | 201.26 | 0.26 | 177.95 | 4.05 | 5.76 |
| | 6.3 | 15 | 2378.84 | 371.94 | 0.16 | 92.26 | 1.37 | 5.44 |
| | | 20 | 1280.51 | 271.50 | 0.21 | 161.15 | 2.22 | 5.42 |
| | | 25 | 823.03 | 219.59 | 0.27 | 121.75 | 3.51 | 5.41 |
| | 6.0 | 15 | 2285.33 | 528.17 | 0.23 | 87.33 | 0.58 | 5.18 |
| | | 20 | 684.38 | 142.19 | 0.21 | 105.26 | 2.68 | 5.16 |
| | | 25 | 464.53 | 122.20 | 0.26 | 89.00 | 4.66 | 5.16 |
| 28 | 7.0 | 20 | 334.89 | 221.65 | 0.66 | 476.65 | 20.93 | 6.18 |
| | | 30 | 195.61 | 143.58 | 0.73 | 297.94 | 13.68 | 6.12 |
| | | 40 | 152.82 | 143.50 | 0.94 | 143.26 | 13.02 | 6.13 |
| | 6.8 | 20 | 938.50 | 261.60 | 0.28 | 258.21 | 3.82 | 5.80 |
| | | 30 | 671.43 | 245.27 | 0.37 | 246.36 | 6.05 | 5.82 |
| | | 40 | 493.99 | 180.09 | 0.36 | 226.40 | 5.74 | 5.78 |
| | 6.5 | 20 | 2301.68 | 443.11 | 0.19 | 181.42 | 0.52 | 5.38 |
| | | 30 | 2004.94 | 337.03 | 0.17 | 233.57 | 1.15 | 5.43 |
| | | 40 | 1809.72 | 295.07 | 0.16 | 221.71 | 1.24 | 5.39 |
| | 6.3 | 20 | 2316.80 | 862.86 | 0.37 | 183.00 | 0.38 | 5.24 |
| | | 30 | 2291.21 | 873.15 | 0.38 | 156.23 | 0.65 | 5.26 |
| | | 40 | 2112.98 | 296.38 | 0.14 | 163.21 | 2.00 | 5.21 |
| | 6.0 | 20 | 1589.59 | 196.53 | 0.12 | 229.91 | 1.30 | 5.03 |
| | | 30 | 1265.95 | 202.85 | 0.16 | 163.55 | 1.47 | 5.05 |
| | | 40 | 1188.48 | 189.05 | 0.16 | 124.42 | 1.00 | 5.04 |

### Example 3: reaction condition: hyaluronic acid concentration 14.5 wt %, 0.58 N HCl, reaction temperature -20°C

The steps of the method and the reaction conditions of Example 3 were the same as those of Example 1, except that the reaction temperature was -20°C, and the reaction time was 14 days, 21 days, 28days, and 35 days.

**Table 3: The reaction conditions and the properties of the auto-crosslinked hyaluronic acid gels which were auto-crosslinked at -20°C in the presence of 0.58 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 44.13 | 40.49 | 0.92 | 38.95 | 3.59 | 6.25 |
| | 6.8 | 10 | 86.01 | 45.54 | 0.53 | 66.02 | 5.68 | 5.92 |
| | 6.5 | 10 | 64.30 | 41.72 | 0.65 | 61.70 | 4.18 | 5.64 |
| | 6.3 | 10 | 47.43 | 32.59 | 0.69 | 52.63 | 4.24 | 5.39 |
| | 6.0 | 10 | 6.13 | 10.13 | 1.65 | 4.80 | 1.23 | 5.06 |
| 21 | 7.0 | 10 | 139.68 | 117.57 | 0.84 | 110.70 | 8.90 | 6.28 |
| | | 15 | 128.13 | 104.58 | 0.82 | 107.57 | 9.62 | 6.31 |
| | 6.8 | 10 | 492.21 | 195.21 | 0.40 | 274.27 | 9.84 | 5.91 |
| | | 15 | 342.42 | 170.51 | 0.50 | 270.02 | 6.01 | 5.86 |
| | 6.5 | 10 | 341.18 | 143.75 | 0.42 | 194.15 | 4.93 | 5.51 |
| | | 15 | 273.02 | 135.30 | 0.50 | 194.43 | 7.81 | 5.50 |
| | 6.3 | 10 | 132.93 | 89.15 | 0.67 | 228.64 | 11.87 | 5.24 |
| | | 15 | 129.90 | 93.09 | 0.72 | 222.74 | 12.18 | 5.22 |
| | 6.0 | 10 | 9.38 | 20.98 | 2.24 | 7.14 | 2.69 | 4.82 |
| | | 15 | 5.22 | 11.06 | 2.12 | 3.65 | 1.95 | 4.79 |
| 28 | 7.0 | 10 | 702.64 | 333.15 | 0.47 | 438.82 | 20.37 | 6.26 |
| | | 15 | 383.58 | 265.95 | 0.69 | 166.92 | 14.45 | 6.25 |
| | | 20 | 214.47 | 241.78 | 1.13 | 131.72 | 13.34 | 6.22 |
| | 6.8 | 10 | 3519.87 | 590.94 | 0.17 | 454.09 | 2.55 | 5.92 |
| | | 15 | 1495.25 | 437.33 | 0.29 | 368.06 | 5.91 | 5.95 |
| | | 20 | 470.11 | 263.71 | 0.56 | 469.35 | 20.27 | 5.93 |
| | 6.5 | 10 | 2075.98 | 467.84 | 0.23 | 349.18 | 4.24 | 5.42 |
| | | 15 | 775.57 | 317.86 | 0.41 | 520.23 | 18.13 | 5.49 |
| | | 20 | 219.25 | 181.55 | 0.83 | 160.71 | 13.29 | 5.50 |
| | 6.3 | 10 | 1997.23 | 488.30 | 0.24 | 315.85 | 4.15 | 5.28 |
| | | 15 | 691.90 | 244.74 | 0.35 | 335.79 | 7.70 | 5.16 |
| | | 20 | 243.58 | 187.81 | 0.77 | 209.24 | 14.23 | 5.18 |
| | 6.0 | 10 | 330.53 | 154.39 | 0.47 | 296.81 | 24.03 | 4.88 |
| | | 15 | 268.58 | 151.83 | 0.57 | 194.00 | 19.70 | 4.79 |
| | | 20 | 12.25 | 33.49 | 2.73 | 7.92 | 3.49 | 4.81 |
| 35 | 7.0 | 10 | 3766.73 | 687.19 | 0.18 | 264.70 | 1.21 | 6.12 |
| | | 35 | 316.12 | 301.37 | 0.95 | 223.59 | 20.19 | 6.10 |
| | 6.8 | 10 | 7999.82 | 1668.22 | 0.21 | 315.46 | 0.69 | 5.98 |
| | | 35 | 1588.83 | 417.33 | 0.26 | 378.94 | 2.17 | 5.72 |
| | 6.5 | 10 | 7898.54 | 1455.39 | 0.18 | 183.33 | 0.63 | 5.74 |
| | | 35 | 977.43 | 441.29 | 0.45 | 554.09 | 3.51 | 5.87 |
| | 6.3 | 10 | 6837.07 | 2231.16 | 0.33 | 97.64 | 0.43 | 5.13 |
| | | 35 | 739.02 | 286.83 | 0.39 | 297.94 | 5.35 | 5.11 |
| | 6.0 | 10 | 988.12 | 327.88 | 0.33 | 323.41 | 6.01 | 4.92 |
| | | 35 | 26.75 | 49.28 | 1.84 | 20.10 | 6.44 | 4.83 |

### Example 4: reaction condition: hyaluronic acid concentration 14.5 wt %, 1.5 N HCl, reaction temperature -20°C

The steps of the method and the reaction conditions of Example 4 were the same as those of Example 1, except that the amount of distilled water was 46.18 g, the amount of 6 N HCl was 21.82 g, and the reaction time was 7 days, 14 days, 21 days, and 28 days.

**Table 4: The reaction conditions and the properties of the auto-crosslinked hyaluronic acid gels which were auto-crosslinked at -20°C and in the presence of 1.5 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 7 | 7.0 | 10 | 82.73 | 66.99 | 0.81 | 107.53 | 6.50 | 6.71 |
| | 6.8 | 10 | 287.91 | 115.26 | 0.40 | 133.78 | 6.22 | 6.31 |
| | 6.5 | 10 | 394.70 | 120.14 | 0.30 | 182.79 | 9.37 | 5.54 |
| | 6.3 | 10 | 171.46 | 68.76 | 0.40 | 76.64 | 8.42 | 5.20 |
| | 6.0 | 10 | 10.76 | 14.83 | 1.38 | 19.34 | 2.06 | 4.79 |
| 14 | 7.0 | 15 | 327.78 | 127.35 | 0.39 | 147.10 | 8.36 | 6.51 |
| | | 20 | 37.69 | 51.57 | 1.37 | 26.05 | 6.29 | 6.45 |
| | 6.8 | 15 | 2084.43 | 399.93 | 0.19 | 216.40 | 2.42 | 6.24 |
| | | 20 | 447.96 | 142.80 | 0.32 | 180.51 | 6.63 | 6.21 |
| | 6.5 | 15 | 3070.94 | 553.37 | 0.18 | 422.89 | 0.97 | 5.98 |
| | | 20 | 583.38 | 125.91 | 0.22 | 201.87 | 5.88 | 5.91 |
| | 6.3 | 15 | 1718.40 | 267.21 | 0.16 | 284.48 | 1.72 | 5.52 |
| | | 20 | 112.55 | 45.79 | 0.41 | 118.27 | 8.09 | 5.44 |
| | 6.0 | 15 | 117.49 | 42.67 | 0.36 | 112.46 | 10.65 | 4.91 |
| | | 20 | 12.29 | 31.46 | 2.56 | 12.84 | 3.61 | 4.95 |
| 21 | 7.0 | 20 | 252.82 | 127.00 | 0.50 | 145.41 | 10.64 | 6.41 |
| | | 30 | 98.14 | 80.32 | 0.82 | 134.28 | 9.63 | 6.45 |
| | 6.8 | 20 | 1459.16 | 281.95 | 0.19 | 198.94 | 0.77 | 6.18 |
| | | 30 | 621.50 | 138.73 | 0.22 | 123.41 | 7.02 | 6.18 |
| | 6.5 | 20 | 1692.62 | 296.59 | 0.18 | 172.78 | 1.56 | 5.41 |
| | | 30 | 894.23 | 202.16 | 0.23 | 181.15 | 5.70 | 5.40 |
| | 6.3 | 20 | 442.16 | 99.76 | 0.23 | 69.02 | 5.17 | 5.07 |
| | | 30 | 116.18 | 50.47 | 0.43 | 31.23 | 4.51 | 5.09 |
| | 6.0 | 20 | 78.47 | 39.96 | 0.51 | 29.39 | 4.71 | 4.78 |
| | | 30 | 25.10 | 53.03 | 2.11 | 14.92 | 5.47 | 4.77 |
| 28 | 7.0 | 30 | 348.80 | 101.52 | 0.29 | 87.45 | 7.87 | 6.25 |
| | | 40 | 37.98 | 72.17 | 1.90 | 25.41 | 7.19 | 6.13 |
| | | 50 | 30.18 | 58.58 | 1.94 | 20.81 | 6.12 | 6.11 |
| | 6.8 | 30 | 1119.54 | 227.50 | 0.20 | 158.47 | 3.96 | 5.95 |
| | | 40 | 179.85 | 73.80 | 0.41 | 68.34 | 9.26 | 5.92 |
| | | 50 | 124.29 | 110.80 | 0.89 | 211.32 | 12.27 | 5.93 |
| | 6.5 | 30 | 1930.83 | 333.35 | 0.17 | 218.70 | 1.17 | 5.30 |
| | | 40 | 418.74 | 135.29 | 0.32 | 121.95 | 10.51 | 5.22 |
| | | 50 | 312.15 | 109.83 | 0.35 | 97.65 | 9.35 | 5.24 |
| | 6.3 | 30 | 534.59 | 210.74 | 0.39 | 176.89 | 7.00 | 4.97 |
| | | 40 | 143.01 | 85.89 | 0.60 | 117.64 | 19.42 | 4.94 |
| | | 50 | 104.47 | 71.90 | 0.69 | 114.23 | 18.23 | 4.96 |
| | 6.0 | 30 | 123.62 | 111.77 | 0.90 | 169.98 | 12.65 | 4.61 |
| | | 40 | 24.41 | 24.98 | 1.02 | 99.54 | 8.49 | 4.60 |
| | | 50 | 22.46 | 23.82 | 1.06 | 102.08 | 7.16 | 4.61 |

### Example 5: reaction condition: hyaluronic acid concentration 14.5 wt %, 1.0 N HCl, reaction temperature -30°C

The steps of the method and the reaction conditions of Example 5 were the same as those of Example 2, except that the reaction temperature was -30°C, and the reaction time was 14 days, 21 days, 28 days, and 35 days.

**Table 5: The reaction conditions and the properties of the auto-crosslinked hyaluronic acid gels which were auto-crosslinked at -30°C and in the presence of 1.0 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 56.66 | 52.08 | 0.92 | 77.49 | 5.33 | 6.49 |
| | 6.8 | 10 | 224.94 | 112.33 | 0.50 | 177.00 | 13.61 | 6.17 |
| | 6.5 | 10 | 328.46 | 158.19 | 0.48 | 256.12 | 12.43 | 5.73 |
| | 6.3 | 10 | 159.19 | 123.01 | 0.77 | 134.34 | 9.97 | 5.19 |
| | 6.0 | 10 | 9.04 | 21.54 | 2.38 | 7.26 | 2.84 | 4.65 |
| 21 | 7.0 | 10 | 256.23 | 156.71 | 0.61 | 324.64 | 23.36 | 6.35 |
| | | 20 | 52.11 | 71.74 | 1.38 | 44.92 | 7.70 | 6.14 |
| | 6.8 | 10 | 1869.39 | 434.60 | 0.23 | 331.45 | 2.17 | 5.75 |
| | | 20 | 601.71 | 293.28 | 0.49 | 429.25 | 6.58 | 5.61 |
| | 6.5 | 10 | 1026.49 | 293.97 | 0.29 | 351.69 | 3.69 | 5.03 |
| | | 20 | 401.17 | 270.07 | 0.67 | 342.23 | 19.44 | 4.97 |
| | 6.3 | 10 | 860.80 | 357.23 | 0.41 | 591.35 | 19.22 | 4.68 |
| | | 20 | 311.38 | 193.68 | 0.62 | 289.99 | 4.07 | 4.65 |
| | 6.0 | 10 | 30.34 | 50.82 | 1.68 | 25.87 | 5.85 | 4.36 |
| | | 20 | 2.27 | 13.89 | 6.12 | 3.02 | 2.49 | 4.36 |
| 28 | 7.0 | 15 | 447.42 | 255.14 | 0.57 | 470.89 | 35.66 | 5.88 |
| | | 20 | 352.04 | 224.06 | 0.64 | 344.47 | 25.84 | 5.95 |
| | | 25 | 55.09 | 79.52 | 1.44 | 48.63 | 8.69 | 5.63 |
| | 6.8 | 15 | 4457.73 | 971.62 | 0.22 | 513.83 | 0.45 | 5.36 |
| | | 20 | 3176.37 | 690.29 | 0.22 | 467.98 | 0.91 | 5.37 |
| | | 25 | 1286.77 | 551.91 | 0.43 | 533.98 | 2.75 | 5.34 |
| | 6.5 | 15 | 2216.69 | 624.15 | 0.28 | 460.07 | 2.95 | 4.81 |
| | | 20 | 2088.59 | 628.69 | 0.30 | 480.91 | 2.32 | 4.79 |
| | | 25 | 1043.51 | 519.98 | 0.50 | 1093.48 | 13.55 | 4.78 |
| | 6.3 | 15 | 1441.05 | 448.53 | 0.31 | 393.01 | 4.04 | 4.60 |
| | | 20 | 988.58 | 351.53 | 0.36 | 323.27 | 5.14 | 4.58 |
| | | 25 | 271.82 | 186.61 | 0.69 | 533.38 | 19.96 | 4.57 |
| | 6.0 | 15 | 42.40 | 73.49 | 1.73 | 34.11 | 9.30 | 4.34 |
| | | 20 | 29.52 | 59.36 | 2.01 | 23.72 | 8.10 | 4.31 |
| | | 25 | 3.18 | 17.08 | 5.37 | 2.25 | 2.07 | 4.29 |
| 35 | 7.0 | 15 | 1985.06 | 519.83 | 0.26 | 425.14 | 3.11 | 5.95 |
| | | 25 | 568.35 | 278.43 | 0.49 | 259.20 | 10.21 | 5.79 |
| | 6.8 | 15 | 7737.11 | 1734.93 | 0.22 | 235.52 | 0.51 | 5.38 |
| | | 25 | 6570.25 | 1212.21 | 0.18 | 330.26 | 0.78 | 5.21 |
| | 6.5 | 15 | 6622.27 | 1426.84 | 0.22 | 221.74 | 0.65 | 4.66 |
| | | 25 | 4472.07 | 850.21 | 0.19 | 466.58 | 0.65 | 4.62 |
| | 6.3 | 15 | 4142.30 | 799.00 | 0.19 | 390.78 | 0.50 | 4.47 |
| | | 25 | 1508.53 | 405.25 | 0.27 | 365.86 | 3.21 | 4.47 |
| | 6.0 | 15 | 227.45 | 170.74 | 0.75 | 517.01 | 22.42 | 4.19 |
| | | 25 | 59.91 | 85.00 | 1.42 | 55.29 | 9.97 | 4.20 |

### Example 6: reaction condition: hyaluronic acid concentration 14.5 wt %, 1.5 N HCl, reaction temperature -30°C

The steps of the method and the reaction conditions of Example 6 were the same as those of Example 4, except that the reaction temperature was -30°C, and the reaction time was 14 days, 21 days, 28 days, and 35 days.

**Table 6: The reaction conditions and the properties of the auto-crosslinked hyaluronic acid gels which were auto-crosslinked at -30°C in the presence of 1.5 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 996.96 | 262.36 | 0.26 | 221.25 | 2.36 | 6.28 |
| | | 15 | 144.76 | 75.87 | 0.52 | 94.89 | 11.83 | 6.32 |
| | | 20 | 83.49 | 83.91 | 1.01 | 76.51 | 8.15 | 6.13 |
| | 6.8 | 10 | 2989.10 | 705.56 | 0.24 | 145.20 | 0.43 | 6.12 |
| | | 15 | 736.05 | 191.03 | 0.26 | 174.51 | 5.10 | 6.08 |
| | | 20 | 334.54 | 117.08 | 0.35 | 130.66 | 7.30 | 5.97 |
| | 6.5 | 10 | 2834.10 | 785.63 | 0.28 | 93.79 | 0.42 | 5.76 |
| | | 15 | 617.79 | 185.65 | 0.30 | 201.06 | 5.89 | 5.74 |
| | | 20 | 236.58 | 98.46 | 0.42 | 118.30 | 8.59 | 5.51 |
| | 6.3 | 10 | 1873.02 | 488.39 | 0.26 | 114.42 | 0.47 | 5.51 |
| | | 15 | 164.76 | 120.73 | 0.73 | 214.57 | 10.61 | 5.51 |
| | | 20 | 86.31 | 49.64 | 0.58 | 67.23 | 10.13 | 5.76 |
| | 6.0 | 10 | 237.60 | 121.32 | 0.51 | 191.21 | 10.05 | 5.25 |
| | | 15 | 62.25 | 82.95 | 1.33 | 43.20 | 7.38 | 5.22 |
| | | 20 | 42.50 | 67.63 | 1.59 | 32.60 | 6.46 | 5.07 |
| 21 | 7.0 | 20 | 588.73 | 241.34 | 0.41 | 301.58 | 3.40 | 6.11 |
| | | 25 | 22.83 | 31.71 | 1.39 | 46.75 | 4.05 | 6.28 |
| | 6.8 | 20 | 4275.23 | 969.40 | 0.23 | 229.11 | 0.40 | 6.32 |
| | | 25 | 877.12 | 242.09 | 0.28 | 243.49 | 4.75 | 6.05 |
| | 6.5 | 20 | 3415.02 | 647.12 | 0.19 | 216.30 | 0.51 | 5.85 |
| | | 25 | 393.58 | 195.08 | 0.50 | 230.33 | 4.69 | 5.80 |
| | 6.3 | 20 | 2944.91 | 571.31 | 0.19 | 295.51 | 1.00 | 5.61 |
| | | 25 | 387.70 | 150.62 | 0.39 | 142.10 | 9.58 | 5.54 |
| | 6.0 | 20 | 443.45 | 155.30 | 0.35 | 178.64 | 13.78 | 5.32 |
| | | 25 | 176.06 | 91.04 | 0.52 | 107.40 | 17.20 | 5.28 |
| 28 | 7.0 | 25 | 439.95 | 235.31 | 0.53 | 340.97 | 5.44 | 6.30 |
| | | 30 | 285.81 | 238.69 | 0.84 | 229.96 | 18.79 | 6.30 |
| | 6.8 | 25 | 2810.48 | 486.93 | 0.17 | 232.03 | 0.41 | 5.94 |
| | | 30 | 1969.05 | 389.87 | 0.20 | 238.98 | 1.61 | 5.95 |
| | 6.5 | 25 | 1665.85 | 322.43 | 0.19 | 251.40 | 1.35 | 5.77 |
| | | 30 | 950.09 | 220.48 | 0.23 | 192.30 | 5.45 | 5.80 |
| | 6.3 | 25 | 1229.22 | 365.81 | 0.30 | 278.48 | 3.17 | 5.45 |
| | | 30 | 753.08 | 276.13 | 0.37 | 325.37 | 2.89 | 5.48 |
| | 6.0 | 25 | 220.42 | 193.50 | 0.88 | 143.75 | 10.12 | 5.06 |
| | | 30 | 53.02 | 56.10 | 1.06 | 149.24 | 7.70 | 5.05 |
| 35 | 7.0 | 30 | 962.64 | 372.28 | 0.39 | 380.14 | 5.44 | 6.08 |
| | | 40 | 593.95 | 358.97 | 0.60 | 896.91 | 20.31 | 6.08 |
| | | 50 | 459.78 | 327.40 | 0.71 | 483.85 | 22.80 | 6.05 |
| | 6.8 | 30 | 3551.09 | 646.11 | 0.18 | 230.02 | 0.41 | 5.82 |
| | | 40 | 3261.01 | 613.95 | 0.19 | 269.76 | 0.57 | 5.83 |
| | | 50 | 2485.86 | 450.87 | 0.18 | 254.68 | 1.15 | 5.80 |
| | 6.5 | 30 | 2819.77 | 552.13 | 0.20 | 323.27 | 0.62 | 5.66 |
| | | 40 | 1980.48 | 388.01 | 0.20 | 296.76 | 1.35 | 5.63 |
| | | 50 | 1385.62 | 296.00 | 0.21 | 220.47 | 3.07 | 5.63 |
| | 6.3 | 30 | 2056.32 | 372.58 | 0.18 | 235.49 | 0.97 | 5.25 |
| | | 40 | 1375.73 | 414.35 | 0.30 | 325.25 | 3.95 | 5.21 |
| | | 50 | 1110.71 | 412.24 | 0.37 | 365.51 | 4.60 | 5.15 |
| | 6.0 | 30 | 749.30 | 205.98 | 0.27 | 184.95 | 8.27 | 4.92 |
| | | 40 | 306.59 | 141.20 | 0.46 | 133.73 | 13.46 | 4.95 |
| | | 50 | 156.71 | 81.03 | 0.52 | 68.68 | 11.44 | 4.95 |

### Example 7: reaction condition: hyaluronic acid concentration 10 wt %, 0.58 N HCl, reaction temperature -10°C

The steps of the method and the reaction conditions of Example 7 were the same as those of Example 1, except that the amount of hyaluronic acid was 8.3 g, the amount of distilled water was 63.27 g, the amount of 6 N HCl is 8.43 g, and the reaction time was 7 days, 14 days, 21 days, and 28 days.

**Table 7: The reaction conditions and the properties of the auto-crosslinked hyaluronic acid gels which were auto-crosslinked at -10°C in the presence of 0.58 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 7 | 7.0 | 10 | 22.37 | 27.60 | 1.23 | 15.24 | 2.48 | 6.32 |
| | 6.8 | 10 | 230.13 | 77.55 | 0.34 | 89.53 | 4.04 | 5.83 |
| | 6.5 | 10 | 185.68 | 60.01 | 0.32 | 60.14 | 6.19 | 5.45 |
| | 6.3 | 10 | 40.19 | 23.89 | 0.59 | 57.49 | 3.17 | 5.12 |
| | 6.0 | 10 | 35.12 | 31.51 | 0.90 | 27.65 | 4.14 | 4.94 |
| 14 | 7.0 | 15 | 120.37 | 73.35 | 0.61 | 57.07 | 6.41 | 6.26 |
| | | 25 | 14.43 | 19.07 | 1.32 | 16.52 | 3.65 | 6.23 |
| | | 35 | 7.38 | 14.90 | 2.02 | 7.84 | 1.93 | 6.19 |
| | 6.8 | 15 | 1939.97 | 534.65 | 0.28 | 213.73 | 1.51 | 5.78 |
| | | 25 | 440.96 | 177.26 | 0.40 | 186.10 | 6.82 | 5.77 |
| | | 35 | 373.29 | 167.01 | 0.45 | 227.97 | 9.37 | 5.75 |
| | 6.5 | 15 | 698.91 | 224.95 | 0.32 | 175.01 | 5.25 | 5.54 |
| | | 25 | 190.84 | 119.82 | 0.63 | 176.26 | 6.87 | 5.48 |
| | | 35 | 150.53 | 87.33 | 0.58 | 90.80 | 5.41 | 5.43 |
| | 6.3 | 15 | 629.25 | 241.45 | 0.38 | 157.16 | 3.26 | 5.21 |
| | | 25 | 402.33 | 169.46 | 0.42 | 233.74 | 4.12 | 5.19 |
| | | 35 | 219.98 | 130.25 | 0.59 | 149.83 | 7.63 | 5.20 |
| | 6.0 | 15 | 450.35 | 193.20 | 0.43 | 282.81 | 6.54 | 4.91 |
| | | 25 | 152.93 | 113.74 | 0.74 | 195.94 | 11.02 | 4.89 |
| | | 35 | 44.66 | 35.45 | 0.79 | 94.78 | 13.14 | 4.73 |
| 21 | 7.0 | 25 | 177.41 | 155.01 | 0.87 | 164.71 | 13.46 | 6.13 |
| | | 35 | 114.29 | 116.97 | 1.02 | 86.18 | 9.70 | 6.12 |
| | 6.8 | 25 | 3029.28 | 590.27 | 0.19 | 324.81 | 0.66 | 5.66 |
| | | 35 | 1685.37 | 343.84 | 0.20 | 285.02 | 2.02 | 5.68 |
| | 6.5 | 25 | 2607.97 | 510.68 | 0.20 | 328.31 | 1.69 | 5.38 |
| | | 35 | 1171.78 | 243.377 | 0.21 | 211.96 | 3.94 | 5.32 |
| | 6.3 | 25 | 749.84 | 269.85 | 0.36 | 301.33 | 8.82 | 5.16 |
| | | 35 | 387.09 | 187.39 | 0.48 | 209.48 | 10.02 | 5.14 |
| | 6.0 | 25 | 472.62 | 140.94 | 0.30 | 152.77 | 10.02 | 4.73 |
| | | 35 | 79.52 | 83.80 | 1.05 | 68.38 | 7.29 | 4.89 |
| 28 | 7.0 | 35 | 430.00 | 178.25 | 0.41 | 253.15 | 7.90 | 6.18 |
| | | 45 | 352.93 | 209.82 | 0.59 | 541.04 | 19.59 | 6.20 |
| | 6.8 | 35 | 2334.21 | 492.38 | 0.21 | 305.92 | 0.76 | 5.66 |
| | | 45 | 1616.80 | 369.69 | 0.23 | 307.65 | 2.88 | 5.65 |
| | 6.5 | 35 | 2225.28 | 435.51 | 0.20 | 221.43 | 1.50 | 5.37 |
| | | 45 | 882.75 | 196.21 | 0.22 | 189.77 | 6.43 | 5.28 |
| | 6.3 | 35 | 426.78 | 132.71 | 0.31 | 126.72 | 9.55 | 5.11 |
| | | 45 | 160.89 | 69.07 | 0.43 | 75.48 | 11.39 | 5.09 |
| | 6.0 | 35 | 317.82 | 96.94 | 0.31 | 108.17 | 9.64 | 4.76 |
| | | 45 | 3.23 | 8.99 | 2.78 | 2.40 | 1.35 | 4.72 |

### Example 8: reaction condition: hyaluronic acid concentration 10 wt %, 1.0 N HCl, reaction temperature -10°C

The steps of the method and the reaction conditions of Example 8 were the same as those of Example 7, except that the amount of distilled water was 57.17 g, the amount of 6 N HCl was 14.53 g, and the reaction time is 14 days, 21 days, and 28 days.

**Table 8: The reaction conditions and the properties of the auto-crosslinked hyaluronic acid gels which were auto-crosslinked at -10°C and in the presence of 1.0 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 118.18 | 33.17 | 0.28 | 48.87 | 3.82 | 6.38 |
| | | 15 | 25.77 | 15.79 | 0.61 | 25.85 | 3.35 | 6.36 |
| | 6.8 | 10 | 998.89 | 234.42 | 0.23 | 81.91 | 2.82 | 5.95 |
| | | 15 | 237.86 | 58.02 | 0.24 | 39.74 | 3.19 | 5.92 |
| | 6.5 | 10 | 794.40 | 143.95 | 0.18 | 69.31 | 2.18 | 5.77 |
| | | 15 | 221.99 | 43.83 | 0.20 | 38.04 | 3.08 | 5.75 |
| | 6.3 | 10 | 505.28 | 108.46 | 0.21 | 74.05 | 4.04 | 5.58 |
| | | 15 | 121.12 | 34.83 | 0.29 | 40.69 | 3.14 | 5.56 |
| | 6.0 | 10 | 184.89 | 38.12 | 0.21 | 41.85 | 2.77 | 5.13 |
| | | 15 | 56.78 | 19.81 | 0.35 | 20.28 | 1.68 | 5.11 |
| 21 | 7.0 | 15 | 467.49 | 70.53 | 0.15 | 55.69 | 4.23 | 6.21 |
| | | 25 | 54.92 | 19.48 | 0.35 | 21.59 | 1.98 | 6.18 |
| | | 35 | 41.10 | 16.21 | 0.39 | 21.15 | 3.69 | 6.15 |
| | 6.8 | 15 | 1637.68 | 284.114 | 0.17 | 84.51 | 0.60 | 5.89 |
| | | 25 | 479.60 | 77.78 | 0.16 | 73.53 | 3.04 | 5.91 |
| | | 35 | 165.79 | 45.26 | 0.27 | 56.84 | 5.57 | 5.87 |
| | 6.5 | 15 | 1369.34 | 203.92 | 0.15 | 95.30 | 1.43 | 5.68 |
| | | 25 | 358.04 | 71.58 | 0.20 | 59.26 | 3.53 | 5.67 |
| | | 35 | 97.31 | 29.97 | 0.31 | 39.80 | 3.82 | 5.57 |
| | 6.3 | 15 | 765.70 | 151.72 | 0.20 | 83.79 | 3.16 | 5.18 |
| | | 25 | 113.67 | 38.64 | 0.34 | 46.75 | 3.81 | 5.16 |
| | | 35 | 24.30 | 18.32 | 0.75 | 44.93 | 4.22 | 5.16 |
| | 6.0 | 15 | 218.44 | 61.25 | 0.28 | 64.71 | 4.48 | 4.91 |
| | | 25 | 22.74 | 19.30 | 0.85 | 71.18 | 4.17 | 4.87 |
| | | 35 | 7.60 | 13.37 | 1.76 | 5.12 | 1.54 | 4.91 |
| 28 | 7.0 | 25 | 79.11 | 22.56 | 0.29 | 59.16 | 6.81 | 6.23 |
| | | 35 | 7.65 | 6.26 | 0.82 | 16.58 | 2.38 | 6.21 |
| | | 45 | 6.83 | 5.67 | 0.83 | 12.38 | 2.88 | 6.21 |
| | 6.8 | 25 | 504.75 | 90.85 | 0.18 | 78.91 | 4.19 | 5.84 |
| | | 35 | 428.34 | 77.02 | 0.18 | 78.00 | 4.93 | 5.81 |
| | | 45 | 107.60 | 32.29 | 0.30 | 53.74 | 4.26 | 5.85 |
| | 6.5 | 25 | 375.96 | 61.79 | 0.16 | 58.98 | 3.84 | 5.54 |
| | | 35 | 290.03 | 50.43 | 0.17 | 69.65 | 4.78 | 5.54 |
| | | 45 | 73.53 | 24.30 | 0.33 | 35.69 | 2.80 | 5.51 |
| | 6.3 | 25 | 146.91 | 47.52 | 0.32 | 54.68 | 4.12 | 5.17 |
| | | 35 | 76.57 | 30.99 | 0.40 | 50.17 | 5.17 | 5.07 |
| | | 45 | 22.64 | 18.10 | 0.80 | 27.21 | 2.50 | 5.12 |
| | 6.0 | 25 | 38.39 | 25.56 | 0.67 | 26.96 | 2.18 | 4.95 |
| | | 35 | 22.12 | 22.83 | 1.03 | 26.77 | 2.79 | 4.82 |
| | | 45 | 6.14 | 10.65 | 1.73 | 4.53 | 1.28 | 4.88 |

### Example 9: reaction condition: hyaluronic acid concentration 10 wt %, 0.58 N HCl, reaction temperature -20°C

The steps of the method and the reaction conditions of Example 9 were the same as those of Example 7, except that the reaction temperature was -20°C, and the reaction time was 7 days, 14 days, 21 days, and 28 days.

**Table 9: The reaction conditions and the properties of the auto-crosslinked hyaluronic acid gels which were auto-crosslinked at -20°C in the presence of 0.58 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 7 | 7.0 | 15 | 860.56 | 151.19 | 0.18 | 522.40 | 22.25 | 6.15 |
| | 6.8 | 15 | 2740.76 | 446.44 | 0.16 | 173.41 | 0.43 | 5.89 |
| | 6.5 | 15 | 2065.98 | 445.28 | 0.22 | 216.40 | 2.42 | 5.62 |
| | 6.3 | 15 | 1510.06 | 348.25 | 0.23 | 107.18 | 4.70 | 5.33 |
| | 6.0 | 15 | 272.63 | 174.79 | 0.64 | 136.13 | 7.80 | 4.85 |
| 14 | 7.0 | 25 | 1407.36 | 448.84 | 0.32 | 303.42 | 5.27 | 6.28 |
| | | 35 | 65.32 | 79.80 | 1.22 | 68.00 | 9.21 | 6.21 |
| | 6.8 | 25 | 9831.20 | 2661.73 | 0.27 | 256.73 | 0.58 | 5.75 |
| | | 35 | 2394.41 | 824.27 | 0.34 | 535.68 | 3.42 | 5.70 |
| | 6.5 | 25 | 8572.12 | 3209.30 | 0.37 | 68.35 | 0.83 | 5.51 |
| | | 35 | 1541.45 | 665.44 | 0.43 | 625.77 | 2.97 | 5.45 |
| | 6.3 | 25 | 6228.97 | 1505.46 | 0.24 | 466.58 | 0.65 | 5.37 |
| | | 35 | 493.57 | 300.73 | 0.61 | 410.75 | 29.04 | 5.32 |
| | 6.0 | 25 | 237.60 | 121.32 | 0.51 | 191.21 | 10.15 | 4.81 |
| | | 35 | 1.66 | 11.86 | 7.14 | 2.28 | 2.19 | 4.79 |
| 21 | 7.0 | 35 | 323.43 | 232.57 | 0.71 | 360.32 | 19.10 | 6.22 |
| | | 45 | 4.62 | 25.33 | 5.48 | 5.15 | 4.26 | 6.21 |
| | 6.8 | 35 | 3188.65 | 596.26 | 0.18 | 220.25 | 0.51 | 5.76 |
| | | 45 | 2321.62 | 1024.15 | 0.44 | 999.43 | 2.14 | 5.76 |
| | 6.5 | 35 | 2421.45 | 492.03 | 0.20 | 274.59 | 0.61 | 5.58 |
| | | 45 | 1118.00 | 577.42 | 0.52 | 726.10 | 5.06 | 5.55 |
| | 6.3 | 35 | 1685.52 | 537.38 | 0.31 | 449.84 | 1.81 | 5.43 |
| | | 45 | 211.85 | 107.32 | 0.51 | 135.67 | 15.55 | 5.41 |
| | 6.0 | 35 | 104.23 | 58.85 | 0.56 | 64.61 | 11.55 | 4.95 |
| | | 45 | 1.07 | 4.30 | 4.02 | 0.72 | 0.78 | 4.92 |
| 28 | 7.0 | 45 | 91.34 | 52.48 | 0.57 | 91.96 | 8.42 | 6.22 |
| | | 60 | 43.55 | 44.44 | 1.02 | 32.04 | 3.87 | 6.17 |
| | 6.8 | 45 | 3176.86 | 589.92 | 0.19 | 422.89 | 0.97 | 5.83 |
| | | 60 | 564.88 | 240.80 | 0.43 | 307.25 | 5.65 | 5.85 |
| | 6.5 | 45 | 1548.54 | 693.96 | 0.45 | 115.38 | 0.31 | 5.43 |
| | | 60 | 243.77 | 165.43 | 0.68 | 296.81 | 24.03 | 5.37 |
| | 6.3 | 45 | 691.63 | 227.43 | 0.33 | 279.56 | 11.88 | 5.24 |
| | | 60 | 135.20 | 94.37 | 0.7 | 93.94 | 7.67 | 5.18 |
| | 6.0 | 45 | 85.24 | 49.09 | 0.58 | 89.70 | 7.84 | 4.86 |
| | | 60 | 36.53 | 47.25 | 1.29 | 22.65 | 3.87 | 4.82 |

### Example 10: reaction condition: hyaluronic acid concentration 10 wt %, 1.0 N HCl, reaction temperature -20°C

The steps of the method and the reaction conditions of Example 10 were the same as those of Example 8, except that the reaction temperature was -20°C.

**Table 10: The reaction conditions and the properties of the auto-crosslinked hyaluronic acid gels which were auto-crosslinked at -20°C in the presence of 1.0 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 7 | 7.0 | 10 | 159.19 | 78.60 | 0.49 | 94.38 | 6.78 | 6.73 |
| | | 15 | 98.29 | 60.74 | 0.62 | 117.69 | 9.48 | 6.73 |
| | 6.8 | 10 | 1760.47 | 377.83 | 0.21 | 175.54 | 2.13 | 6.47 |
| | | 15 | 1017.05 | 250.62 | 0.25 | 145.35 | 3.73 | 6.46 |
| | 6.5 | 10 | 1122.78 | 231.97 | 0.21 | 159.85 | 3.98 | 5.87 |
| | | 15 | 634.43 | 146.76 | 0.23 | 119.36 | 6.04 | 5.86 |
| | 6.3 | 10 | 708.22 | 246.12 | 0.35 | 152.91 | 5.63 | 5.44 |
| | | 15 | 390.24 | 109.80 | 0.28 | 121.23 | 7.00 | 5.39 |
| | 6.0 | 10 | 228.96 | 73.44 | 0.32 | 75.38 | 6.21 | 4.96 |
| | | 15 | 91.04 | 38.63 | 0.42 | 40.71 | 4.85 | 4.97 |
| 14 | 7.0 | 20 | 844.97 | 297.81 | 0.35 | 285.46 | 6.40 | 6.52 |
| | | 30 | 102.68 | 112.23 | 1.09 | 77.50 | 9.30 | 6.57 |
| | 6.8 | 20 | 4054.92 | 1240.28 | 0.31 | 135.82 | 0.39 | 6.35 |
| | | 30 | 1926.62 | 431.78 | 0.22 | 203.67 | 1.02 | 6.45 |
| | 6.5 | 20 | 3371.48 | 1104.07 | 0.33 | 96.95 | 0.50 | 5.63 |
| | | 30 | 1906.36 | 411.25 | 0.22 | 207.00 | 0.85 | 5.68 |
| | 6.3 | 20 | 2585.68 | 569.96 | 0.22 | 228.56 | 0.34 | 5.25 |
| | | 30 | 378.76 | 113.29 | 0.30 | 98.69 | 7.24 | 5.27 |
| | 6.0 | 20 | 2355.13 | 544.61 | 0.23 | 183.34 | 0.68 | 4.79 |
| | | 30 | 283.05 | 161.43 | 0.57 | 309.74 | 10.78 | 4.79 |
| 21 | 7.0 | 30 | 131.81 | 119.29 | 0.91 | 123.05 | 11.27 | 6.48 |
| | | 40 | 74.89 | 88.16 | 1.18 | 59.18 | 8.01 | 6.56 |
| | | 50 | 40.89 | 62.40 | 1.53 | 28.25 | 5.88 | 6.57 |
| | 6.8 | 30 | 3142.40 | 828.70 | 0.26 | 203.94 | 0.72 | 6.31 |
| | | 40 | 3080.07 | 664.24 | 0.22 | 252.88 | 0.83 | 6.30 |
| | | 50 | 1472.10 | 331.27 | 0.23 | 246.17 | 3.59 | 6.28 |
| | 6.5 | 30 | 3141.68 | 776.34 | 0.25 | 197.29 | 0.40 | 5.63 |
| | | 40 | 2764.63 | 654.75 | 0.24 | 212.69 | 0.57 | 5.59 |
| | | 50 | 904.23 | 235.98 | 0.26 | 153.64 | 4.91 | 5.53 |
| | 6.3 | 30 | 614.25 | 127.18 | 0.21 | 107.73 | 7.23 | 5.14 |
| | | 40 | 471.43 | 113.44 | 0.24 | 94.44 | 6.86 | 5.15 |
| | | 50 | 416.70 | 100.48 | 0.24 | 98.60 | 7.85 | 5.13 |
| | 6.0 | 30 | 431.49 | 157.11 | 0.36 | 161.09 | 8.29 | 4.69 |
| | | 40 | 393.78 | 161.98 | 0.41 | 180.99 | 9.58 | 4.68 |
| | | 50 | 3.90 | 8.84 | 2.27 | 3.84 | 1.40 | 4.42 |
| 28 | 7.0 | 50 | 314.41 | 109.60 | 0.35 | 111.58 | 14.86 | 5.75 |
| | | 60 | 46.22 | 37.02 | 0.80 | 106.24 | 10.99 | 5.79 |
| | 6.8 | 50 | 2679.42 | 595.86 | 0.22 | 272.77 | 0.84 | 5.46 |
| | | 60 | 430.23 | 158.95 | 0.37 | 160.24 | 9.96 | 5.51 |
| | 6.5 | 50 | 2206.18 | 473.56 | 0.21 | 230.36 | 0.70 | 4.96 |
| | | 60 | 426.79 | 155.27 | 0.36 | 153.87 | 14.45 | 4.97 |
| | 6.3 | 50 | 730.25 | 306.71 | 0.42 | 334.62 | 8.19 | 4.69 |
| | | 60 | 212.40 | 145.04 | 0.68 | 443.00 | 21.19 | 4.66 |
| | 6.0 | 50 | 186.29 | 113.61 | 0.61 | 241.52 | 19.83 | 4.42 |
| | | 60 | 0.76 | 2.74 | 3.61 | 0.51 | 0.44 | 4.42 |

### Example 11: reaction condition: hyaluronic acid concentration 10 wt %, 0.58 N HCl, reaction temperature -30°C

The steps of the method and the reaction conditions of Example 11 were the same as those of Example 7, except that the reaction temperature was -30°C, and the reaction time was 14 days, 21 days, 28 days, and 35 days.

**Table 11: The reaction conditions and the properties of the auto-crosslinked hyaluronic acid gels which were auto-crosslinked at -30°C in the presence of 0.58 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 10 | 124.91 | 89.56 | 0.72 | 98.35 | 6.72 | 6.21 |
| | 6.8 | 10 | 653.20 | 198.14 | 0.30 | 199.36 | 4.63 | 5.82 |
| | 6.5 | 10 | 510.96 | 179.83 | 0.35 | 195.01 | 5.85 | 5.58 |
| | 6.3 | 10 | 209.10 | 104.92 | 0.50 | 208.67 | 8.30 | 5.25 |
| | 6.0 | 10 | 82.08 | 69.42 | 0.85 | 140.94 | 8.03 | 5.06 |
| 21 | 7.0 | 10 | 586.43 | 248.74 | 0.42 | 357.38 | 3.11 | 6.35 |
| | | 15 | 133.74 | 100.93 | 0.75 | 177.57 | 8.99 | 6.29 |
| | 6.8 | 10 | 2109.86 | 565.40 | 0.27 | 330.26 | 0.78 | 5.87 |
| | | 15 | 436.65 | 210.33 | 0.48 | 348.79 | 7.06 | 5.81 |
| | 6.5 | 10 | 1033.33 | 388.76 | 0.38 | 399.80 | 2.90 | 5.52 |
| | | 15 | 264.48 | 200.93 | 0.76 | 190.32 | 13.07 | 5.45 |
| | 6.3 | 10 | 786.11 | 255.03 | 0.32 | 437.95 | 14.95 | 5.14 |
| | | 15 | 390.86 | 221.20 | 0.57 | 253.76 | 4.46 | 5.15 |
| | 6.0 | 10 | 103.56 | 87.15 | 0.84 | 142.38 | 8.36 | 5.03 |
| | | 15 | 19.23 | 30.71 | 1.60 | 14.57 | 3.24 | 5.07 |
| 28 | 7.0 | 10 | 620.01 | 231.00 | 0.37 | 277.52 | 14.88 | 6.30 |
| | | 15 | 184.38 | 142.87 | 0.77 | 220.72 | 6.70 | 6.28 |
| | | 20 | 53.97 | 65.47 | 1.21 | 50.11 | 6.73 | 6.11 |
| | 6.8 | 10 | 3135.03 | 919.02 | 0.29 | 300.67 | 0.87 | 6.05 |
| | | 15 | 1301.66 | 457.69 | 0.35 | 449.08 | 3.21 | 6.03 |
| | | 20 | 364.36 | 212.06 | 0.58 | 425.23 | 16.60 | 5.96 |
| | 6.5 | 10 | 2980.60 | 819.94 | 0.28 | 348.33 | 0.74 | 5.75 |
| | | 15 | 1167.22 | 522.60 | 0.45 | 734.55 | 3.49 | 5.74 |
| | | 20 | 285.84 | 169.04 | 0.59 | 292.76 | 19.07 | 5.61 |
| | 6.3 | 10 | 1252.84 | 374.26 | 0.30 | 337.75 | 2.91 | 5.48 |
| | | 15 | 616.14 | 287.60 | 0.47 | 455.96 | 5.90 | 5.42 |
| | | 20 | 168.44 | 162.95 | 0.97 | 121.04 | 11.35 | 5.36 |
| | 6.0 | 10 | 151.45 | 87.22 | 0.58 | 172.69 | 10.17 | 5.12 |
| | | 15 | 21.10 | 40.31 | 1.91 | 14.74 | 4.89 | 4.88 |
| | | 20 | 2.37 | 10.63 | 4.49 | 2.44 | 1.71 | 4.92 |
| 35 | 7.0 | 20 | 55.35 | 72.04 | 1.30 | 48.77 | 7.32 | 5.91 |
| | 6.8 | 20 | 733.79 | 418.38 | 0.57 | 729.49 | 23.14 | 5.74 |
| | 6.5 | 20 | 409.94 | 241.02 | 0.59 | 443.69 | 18.28 | 5.57 |
| | 6.3 | 20 | 323.39 | 181.68 | 0.56 | 286.95 | 19.17 | 5.15 |
| | 6.0 | 20 | 5.44 | 18.58 | 3.42 | 4.36 | 2.95 | 4.82 |

### Example 12: reaction condition: hyaluronic acid concentration 10 wt %, 1.0 N HCl, reaction temperature -30°C

The steps of the method and the reaction conditions of Example 12 were the same as those of Example 8, except that the reaction temperature was -30°C, and the reaction time was 14 days, 21 days, and 28 days.

**Table 12: The reaction conditions and the properties of the auto-crosslinked hyaluronic acid gels which were auto-crosslinked at -30°C and in the presence of 1.0 N HCl.**

| Reaction time (day) | pH before steam treatment | 121°C steam treatment time (min) | G'(Pa) (25°C, 1Hz) | G"(Pa) (25°C, 1Hz) | tanδ (25°C, 1Hz) | Shear viscosity (Pa·s), 25°C | | Gel pH |
|---|---|---|---|---|---|---|---|---|
| | | | | | | 1 sec⁻¹ | 50 sec⁻¹ | |
| 14 | 7.0 | 15 | 1845.20 | 513.17 | 0.28 | 514.90 | 3.53 | 6.24 |
| | 6.8 | 15 | 4451.31 | 968.10 | 0.22 | 514.18 | 0.58 | 5.93 |
| | 6.5 | 15 | 1736.48 | 409.25 | 0.24 | 406.89 | 2.56 | 5.61 |
| | 6.3 | 15 | 481.41 | 202.82 | 0.42 | 186.79 | 10.10 | 5.12 |
| | 6.0 | 15 | 69.21 | 72.52 | 1.05 | 89.96 | 7.57 | 4.87 |
| 21 | 7.0 | 25 | 993.47 | 408.34 | 0.41 | 458.27 | 6.73 | 6.25 |
| | 6.8 | 25 | 1818.42 | 446.43 | 0.25 | 440.00 | 1.23 | 5.91 |
| | 6.5 | 25 | 607.28 | 228.92 | 0.38 | 223.54 | 8.59 | 5.52 |
| | 6.3 | 25 | 120.79 | 92.68 | 0.77 | 275.35 | 17.42 | 5.17 |
| | 6.0 | 25 | 2.87 | 11.01 | 3.84 | 2.22 | 1.56 | 4.94 |
| 28 | 7.0 | 25 | 3693.45 | 918.54 | 0.25 | 652.74 | 0.59 | 6.15 |
| | 6.8 | 25 | 6847.48 | 138.46 | 0.02 | 572.23 | 0.27 | 5.89 |
| | 6.5 | 25 | 1820.11 | 461.03 | 0.25 | 384.62 | 2.25 | 5.51 |
| | 6.3 | 25 | 378.36 | 176.58 | 0.47 | 140.52 | 14.13 | 5.17 |
| | 6.0 | 25 | 22.80 | 40.43 | 1.77 | 18.24 | 5.01 | 4.92 |

The physical properties of Examples 1 to 12 are shown in Table 13 and FIGs. 1 to 5.

**Table 13: The reaction conditions and the properties of the auto-crosslinked hyaluronic acid gels of Examples 1 to 12.**

| Examples | HA concentration in mixture (wt%) | Reaction temperature (°C) | HCl concentration in mixture (N) | Reaction time (day) | Equilibrium swelling capacity* | HA content in the auto-crosslinked HA particles (wt%)** | HA recovery rate (%)*** |
|---|---|---|---|---|---|---|---|
| 1 | 14.5 wt% | -10 | 0.58 | 14 | 20.16 | 3.11 | 79.68 |
| | | | | 21 | 15.82 | 4.35 | 87.34 |
| | | | | 28 | 14.12 | 5.38 | 95.02 |
| | | | | 35 | 12.83 | 6.52 | 99.22 |
| | | | | 42 | 12.07 | 7.57 | 99.82 |
| 2 | 14.5 wt% | -10 | 1.0 | 14 | 19.69 | 3.24 | 82.87 |
| | | | | 21 | 15.43 | 4.42 | 88.25 |
| | | | | 28 | 13.93 | 5.43 | 96.54 |
| 3 | 14.5 wt% | -20 | 0.58 | 14 | 28.77 | 1.41 | 76.48 |
| | | | | 21 | 22.24 | 2.66 | 83.84 |
| | | | | 28 | 17.63 | 3.88 | 91.76 |
| | | | | 35 | 15.03 | 4.09 | 98.70 |
| 4 | 14.5 wt% | -20 | 1.5 | 7 | 25.71 | 2.19 | 88.70 |
| | | | | 14 | 21.28 | 2.88 | 91.65 |
| | | | | 21 | 17.89 | 3.55 | 93.56 |
| | | | | 28 | 14.30 | 5.12 | 95.86 |
| 5 | 14.5 wt% | -30 | 1.0 | 14 | 25.26 | 2.23 | 70.21 |
| | | | | 21 | 18.17 | 3.31 | 76.06 |
| | | | | 28 | 15.76 | 4.23 | 78.33 |
| | | | | 35 | 14.05 | 5.17 | 86.56 |
| 6 | 14.5 wt% | -30 | 1.5 | 14 | 22.86 | 2.61 | 80.60 |
| | | | | 21 | 18.34 | 3.54 | 81.92 |
| | | | | 28 | 15.56 | 4.51 | 93.39 |
| | | | | 35 | 13.87 | 5.79 | 96.87 |
| 7 | 10 wt% | -10 | 0.58 | 7 | 28.02 | 1.59 | 75.43 |
| | | | | 14 | 21.38 | 2.98 | 86.58 |
| | | | | 21 | 17.83 | 3.60 | 91.68 |
| | | | | 28 | 16.96 | 3.99 | 98.35 |
| 8 | 10 wt% | -10 | 1.0 | 14 | 31.42 | 1.06 | 83.67 |
| | | | | 21 | 28.59 | 1.56 | 87.26 |
| | | | | 28 | 27.34 | 1.70 | 94.02 |
| 9 | 10 wt% | -20 | 0.58 | 7 | 22.91 | 2.26 | 85.61 |
| | | | | 14 | 14.92 | 4.34 | 88.84 |
| | | | | 21 | 13.47 | 5.28 | 95.74 |
| | | | | 28 | 12.58 | 6.22 | 98.69 |
| 10 | 10 wt% | -20 | 1.0 | 7 | 26.27 | 1.92 | 83.87 |
| | | | | 14 | 18.69 | 3.47 | 95.34 |
| | | | | 21 | 17.51 | 3.81 | 98.61 |
| | | | | 28 | 14.16 | 4.86 | 99.32 |
| 11 | 10 wt% | -30 | 0.58 | 14 | 19.86 | 3.09 | 93.51 |
| | | | | 21 | 15.45 | 4.24 | 98.58 |
| | | | | 28 | 13.61 | 5.24 | 98.23 |
| | | | | 35 | 12.84 | 6.11 | 100.78 |
| 12 | 10 wt% | -30 | 1.0 | 14 | 22.64 | 2.42 | 83.46 |
| | | | | 21 | 15.88 | 3.85 | 90.83 |
| | | | | 28 | 14.68 | 4.68 | 98.35 |

| | | | | | | | |
|---|---|---|---|---|---|---|---|
| ^{∗}: The auto-crosslinked hyaluronic acid particles at pH 7 were soaked in a pH 6.0 buffer solution containing 0.9 wt % sodium chloride and 10 mM sodium phosphate at 5°C for 24 hours to swell to an equilibrium state, and then dried at 25°C to a constant weight. The equilibrium swelling capacity was calculated according to the definition of the present disclosure. ^{∗∗}: The listed HA content in the auto-crosslinked HA particles is an average of the values measured at different pHs before steam treatment (pH 6.0, 6.3, 6.5, 6.8 and 7.0). ^{∗∗∗}: The HA recovery rate is calculated using the average HA content in the auto-crosslinked HA particles. | | | | | | | |

### Example 13: Sample preparation and process of animal tests

Male Wistar rats weighted about 360 g to 390 g were held in quarantine for 7 days. The room temperature was controlled at 24°C, the humidity was maintained at 60% to 70%, and the photoperiod is set at 12 hr light/12 hr dark. The rats were housed in acrylic cages and allowed free access to feed and water. The rats were anesthetized with isoflurane, and the instruments used throughout the surgery were heat-sterilized in order to prevent the rats from dying of post-operative infections. All animal experiments were conducted in accordance with the American Physiological Society Guidelines for the Care and Use of Animals.

The animal test model generally followed the method used by Spicer et al. ("Evaluation of bone regeneration using the rat critical size calvarial defect", Nature Protocols, vol. 7, no. 10, pp. 1918-1929 (2012)) and Chen et al. ("Bone Formation Using Cross-Linked Chitosan Scaffolds in Rat Calvarial Defects ", Implant Dent. vol 27, no. 6, pp. 1-7 (2017)). First, the rat was anesthetized with the gas anesthetic isoflourane and then shaved. The surgical area was disinfected with povidone-iodine alcoholic solution and 70% alcohol, and then injected with ampicillin. Then, the head of the rat was covered with a sterilized tissue paper having a cut hole for exposing only the surgical area. The skin and periosteum just above the head were incised to a size of 1.5 centimeters. The skull was separate from the periosteum by using a periosteal peeler. A 5 mm diameter bone defect was created in the center of the medial sagittal suture (a bulge in the center of the skull, flanked by the parietal bones) by using a 5 mm diameter trephine bur. Then, 0.15 g to 0.2 g of linear hyaluronic acid of 2.05 wt% (control group), the auto-crosslinked hyaluronic acid gels with different hyaluronic acid contents (experimental groups 1 to 4), or CoreBone 1000 bone meal (positive control group) was implanted to the bone defect, and the bone defect was then covered with a Bio-Gide membrane (3M, Geistlich Bio-Oss^{®}, Switzerland). For blank group, the bone defect is directly covered with a Bio-Gide membrane. If the animal waked up from anesthesia during the surgery, the anesthesia were maintained by inhalation of the anesthesia gas from a gas anesthesia machine through a breathing mask. The preparation conditions and properties of the animal test samples are as shown in Table 14.

**Table 14: The reaction conditions and the properties of the auto-crosslinked hyaluronic acid gels for animal tests.**

| | | Control group (linear HA) | Experimental group 1 | Experimental group 2 | Experimental group 3 | Experimental group 4 |
|---|---|---|---|---|---|---|
| HA content (wt%) | | 2.05 | 1.26 | 2.38 | 4.10 | 7.71 |
| pH of the gel after steam treatment | | 5.58 | 5.92 | 5.86 | 5.87 | 5.32 |
| G'(Pa) of the gel after steam treatment (25°C, 1 Hz) | | 123.98 | 86.01 | 342.42 | 348.96 | 883.13 |
| G"(Pa) of the gel after steam treatment (25°C, 1 Hz) | | 128.15 | 45.54 | 170.51 | 251.75 | 442.08 |
| Shear viscosity (Pa·s) (25°C, shear rate 1 sec⁻¹) | | 63.41 | 66.02 | 270.02 | 377.25 | 553.64 |
| Reaction temperature | | NA | -20°C | -20°C | -20°C | -10°C |
| Reaction time | | NA | 14 days | 21 days | 35 days | 42 days |
| HA concentration in mixture | | NA | 14.5 wt% | 14.5 wt% | 14.5 wt% | 14.5 wt % |
| Acid concentration in mixture | | NA | 0.58N HCl | 0.58N HCl | 0.58N HCl | 0.58N HCl |
| Steam treatment condition | PH of auto-crosslinked HA particles before steam treatment | NA | pH 6.8 | pH 6.8 | pH 6.8 | pH 6.5 |
| | Temperature and time of steam treatment | 121°C 10 mins | 121°C 10 mins | 121°C 15 mins | 121°C 55 mins | 121°C 55 mins |

The rat was sacrificed at three months after the experiment. After deep anesthesia, the skull bone was removed from the rat. The sample was first immersed in 10 % paraformaldehyde for four days for fixing, and then sent to Taiwan Mouse Clinic (TMC) for scanning and analysis using micro-computed tomography (micro-CT) 3D imaging system (SkyScan 1076, Bruker, Antwerp, Belgium). The parameters were set as Image Pixel Size (um)=9, Source Voltage (kV)=70, Source Current (uA)=200, Filter=Al 0.5 mm, Exposure (ms)=453, Rotation Step (deg)=0.2, and Region of Interest (ROI): diameter of 5.5 mm × height of 0.5 mm. The micro-CT images were analyzed using sagittal tomography and histomorphometry. The data was evaluated by the newly formed bone volume/total volume (BV/TV (%)) to quantify the amount of bone regeneration.

The results are shown in FIG. 6. The blank group (No. 1) and the control group (No. 2) had the lowest percentages of newly formed bone at 21% and 40%, respectively, while the percentages of newly formed bone of the experimental groups 1 to 4 (Nos. 3 to 6) were 48%, 61%, 49% and 57%, respectively, and the percentage of newly formed bone of the positive control group (No. 7) was 45%. The data showed that the auto-crosslinked hyaluronic acid gel of the present disclosure has a better function of bone regeneration than the commercially available products (the positive control group), linear hyaluronic acid gel (the control group) and the blank group.

While the present disclosure has been described and illustrated with reference to specific embodiments thereof, these descriptions and illustrations are not limiting. It should be understood by those skilled in the art that various changes may be made and equivalents may be substituted without departing from the true spirit and scope of the present disclosure as defined by the appended claims.

## Claims

1. A method for manufacturing an auto-crosslinked hyaluronic acid gel, comprising steps of:
(a) providing a colloid containing hyaluronic acid or a metal salt thereof;
(b) conducting an auto-crosslinking reaction of the colloid containing hyaluronic acid or a metal salt thereof in an acidic environment at about -10°C to about -30°C for about 7 days to about 42 days; and
(c) treating the product of step (b) by steam to obtain the auto-crosslinked hyaluronic acid gel.

2. The method of Claim 1, which is free of using any crosslinking agents and 2-chloro-1-methylpyridinium iodide (CMPI).

3. The method of Claim 1 or 2, wherein step (a) comprises providing the colloid containing hyaluronic acid or a metal salt thereof in a granular form, a stripe form or a string form.

4. The method of any one of Claims 1 to 3, wherein in step (b), the acidic environment is provided by an acid at a concentration of about 0.5 N to about 1.5 N, and the acid is selected from the group consisting of hydrochloric acid, nitric acid and sulfuric acid.

5. The method of any one of Claims 1 to 4, wherein step (b) comprises mixing the colloid containing hyaluronic acid or a metal salt thereof with an acid at room temperature to form a mixture, and letting the mixture stand at about -10°C to about -30°C for about 7 days to about 42 days.

6. The method of Claim 5, wherein a concentration of hyaluronic acid or a metal salt thereof in the mixture is about 10 wt% to about 30 wt%.

7. The method of any one of Claims 1 to 6, wherein in step (b):
the acidic environment is provided by an acid at a concentration of about 0.58 N to about 1.0 N,
and the auto-crosslinking reaction is conducted at about -10°C for about 7 days to about 42 days;
the acidic environment is provided by an acid at a concentration of about 0.58 N to about 1.5 N,
and the auto-crosslinking reaction is conducted at about -20°C for about 7 days to about 35 days;
the acidic environment is provided by an acid at a concentration of about 0.58 N to about 1.0 N,
and the auto-crosslinking reaction is conducted at about -20°C for about 7 days to about 28 days;
the acidic environment is provided by an acid at a concentration of about 1.0 N to about 1.5 N,
and the auto-crosslinking reaction is conducted at about -30°C for about 14 days to about 35 days; or
the acidic environment is provided by an acid at a concentration of about 0.58 N to about 1.0 N,
and the auto-crosslinking reaction is conducted at about -30°C for about 14 days to about 35 days.

8. The method of any one of Claims 1 to 7, which comprises conducting the auto-crosslinking reaction only once, and the entire auto-crosslinking reaction is conducted continuously at about -10°C to about -30°C.

9. The method of any one of Claims 1 to 8, wherein before step (c), the method further comprises homogenizing and/or washing the product of step (b).

10. The method of any one of Claims 1 to 9, wherein the product of step (b) has an equilibrium swelling capacity of about 12 to about 30.

11. The method of any one of Claims 1 to 10, wherein before step (c), the method further comprises adjusting a pH of the product of step (b) to about 6.0 to about 7.0.

12. The method of Claim any one of Claims 1 to 11, wherein step (c) comprises treating the product of step (b) by steam at about 110°C to about 130°C for about 10 minutes to about 60 minutes.

13. An auto-crosslinked hyaluronic acid gel, wherein a tan δ of the auto-crosslinked hyaluronic acid gel at a concentration of about 1.5 wt% to about 7.5 wt% measured at 25°C and at each and every frequency between 0.1 Hz to 10.0 Hz is equal to or less than 1, and the auto-crosslinked hyaluronic acid gel is sterile.

14. The auto-crosslinked hyaluronic acid gel of Claim 13, which exhibits at least one of:
a modulus of elasticity of the auto-crosslinked hyaluronic acid gel at a concentration of about 1.5 wt% to about 7.5 wt% measured at 25°C and at 1.0 Hz is about 30 Pa to about 8000 Pa;
a modulus of viscosity of the auto-crosslinked hyaluronic acid gel at a concentration of about 1.5 wt% to about 7.5 wt% measured at 25°C and at 1.0 Hz is about 20 Pa to about 2000 Pa;
a shear viscosity of the auto-crosslinked hyaluronic acid gel at a concentration of about 1.5 wt% to about 7.5 wt% measured at 25°C and at a shear rate of 1 sec⁻¹ is about 20 Pa·s to about 900 Pa·s; and
a shear viscosity of the auto-crosslinked hyaluronic acid gel at a concentration of about 1.5 wt% to about 7.5 wt% measured at 25°C and at a shear rate of 50 sec⁻¹ is about 0.31 Pa·s to about 35.66 Pa·s.

15. The auto-crosslinked hyaluronic acid gel of Claim 13 or 14 for use in promoting bone regeneration, treating bone defects, preventing post-operative adhesion, subcutaneous filling and/or treating arthropathy.
